# EUROPEAN PATENT APPLICATION

(11) **EP 2 314 714 A1**
(43) Date of publication of application: **27.04.2011**
(21) Application number: 09252465.1
(22) Date of filing: 22.10.2009
(51) Int. Cl.: C12Q 1/68

(54) **Disease susceptibility**

(71) Applicant: Universiteit Leiden, 2311 EZ Leiden (NL)
(72) Inventor: Klok, Melanie Diane, 2333 CC Leiden (NL); De Rijk, Rolandus Hendrikus, 2333 CC Leiden (NL); De Kloet, E Ronald, 2333 CC Leiden (NL)
(74) Representative: Miles, John Stephen

(57) **Abstract**

The invention provides a method of assessing the susceptibility of a subject to an anxiety disorder or depression, the method comprising determining whether the subject has a haplotype comprising rs3216799, rs6814934, rs765e048, rs2070950 and rs2070951 with respective alleles '+CT', 'C', 'T', 'C' and 'C'.

The invention provides a kit of parts or solid substrate for use in assessing the susceptibility of a subject to an anxiety disorder or depression, the kit comprising or the solid substrate having attached thereto one or more nucleic acid molecules that hybridise selectively to a genomic region encompassing any two or more SNPs selected from the group consisting of r53218799, rs6814934, rs7658048, rs2070a50 and rs2070951, and/or that hybridise selectively to a genomic region encompassing two or more polymorphic sites in linkage disequilibrium with any one or more SNPs selected from the group consisting of rs3216799, rs6814934, rs7658048, rs2070950 and rs2070951.

## Description

The present invention relates to a method of determining disease susceptibility. In particular, it relates to a method of determining susceptibility to an anxiety disorder or depression.

Anxiety disorders are common psychiatric disorders which can be classified into the following categories: substance-induced anxiety disorder, generalised anxiety, panic disorder, acute stress disorder, post-traumatic stress disorder, adjustment disorder with anxious features, social phobia, obsessive-compulsive disorder and specific phobias (American Psychiatric Association. Diagnostic and Stafistical Manual of Mental Disorders. 4th ed. Text Revision, Washington, DC: American Psychiatric Association; 2000). Generally, the disorders are chronic conditions which may be present from an early age or they may be initiated by a particular event. The disorders are often triggered by periods of high stress and are frequently accompanied by physiological symptoms such as headache, sweating, muscle spasms, palpitations and hypertension, which may lead to fatigue or even exhaustion.

Anxiety disorders are commonly comorbid with other mental health diseases. Of particular note, depression is believed to occur in as many as 60% of people with anxiety disorders (Cameron, 2007 Psychiatric Times 24(14)). Major depression Is among the most important mental health problems and affects 1-3% of elderly people, whereas 8-25% have minor depression. Depressive symptoms are associated with future impairments in mobility and functioning, and with higher medical costs (Glitay et al, 2006 J Aff Disord 91: 45-52).

The impact of comorbid anxiety and depression is substantial. As demonstrated by the Global Burden of Disease study, neuropsychiatric disorders accounted for more than 13% of all medical disability worldwide and for more than 27% of all noncommunicable disease in 2005 (Cameron, 2007 Psychiatric Times 24(14)). Depression alone produced 10% to 12% of all disability from noncommunicable disease and approximately 5% of all disability (noncommunicable, communicable, injury). Thus, comorbid anxiety and depression may account for as much as 2% to 4% of all medical disability worldwide. In addition, depression (and, thus, comorbid depression and anxiety) is associated with other psychiatric and nonpsychiatric medical conditions (eg, cardiovascular disease, diabetes, HIV/AIDS, maternal and reproductive-related syndromes, and psychosomatic illnesses), with their resulting socioeconomic costs. Taken together, it is clearly important to establish risk factors of anxiety disorders and depression, and particularly ones which do not rely of the subjectiveness of questionnaire based diagnoses.

Further, many patients suffering from anxiety disorders and depression show insufficient treatment responses, and treatment efficacy among patients is very diverse. Moreover, serious side effects may occur before a treatment response observed, which may itself take months. Thus, there is also need for a biomarker that predicts treatment efficacy of anxiety disorders end depression.

Studies conducted by the present inventors have now identified particular variants in the mineralocorticoid receptor (MR) gene that may be used to predict susceptibility to an anxiety disorder or depression, and also treatment efficacy. In one study of 450 elderly subjects, the inventors demonstrated an association between each of five single nuclear polymorphisms (SNPs) in the MR gene, and dispositonal optimism, a stable personality trait believed to confer resilience against depression (Plomin et al, 1992, Person individ Diff 13(8):921-930; and Giltay et al, 2006, J Aff Disord 91:45-52). An association between a haplotype comprising these SNPs and each of dispositional optimism and anxiety (Hospital anxiety and depression scale; HADS-A) was also found. In a second study of 154 students, the Inventors correlated the presence of the same haplotype with reduced symptoms of depression.

The listing or discussion of an apparently prior-published document in this specification should not necessarily be taken as an acknowledgement that the document is part of the state of the art or is common general knowledge.

A first aspect of the invention provides a method of assessing the susceptibility of a subject to an anxiety disorder or depression, the method comprising genotyping any one or more single nucleotide polymorphisms (SNPs) selected from the group consisting of rs3216799, rs6814934, rs7658048, rs2070950 and rs2070951, and/or one or more polymorphic sites which are in linkage disequilibrium with any one or more SNPs selected from the group consisting of rs3216799, rs6814934, rs7658048, rs2070950 and rs207D951, wherein reduced susceptibility is indicated when the allele of the one or more SNPs is respectively one or more of '+CT', 'C', 'T', 'C' and 'C', and/or when the allele of the one or more polymorphic sites Is one that is in linkage disequilibrium with the respective one or more '+CT', 'C', T', 'C' and 'C' alleles of the one or more SNPs.

By 'assessing the susceptibility of a subject to an anxiety disorder or depression' we indude the meaning of assessing the risk of development of an anxiety disorder or depression in a subject. However, it will be appreciated that the method may also be useful in aiding diagnosis of an anxiety disorder or depression.

By 'anxiety disorder' we include any of substanoe-induced anxiety disorder, generalised anxiety, panic disorder, acute stress disorder, post-traumatic stress disorder, adjustment disorder with anxious features, social phobia, obsessive-compulsive disorder or specific phobias.

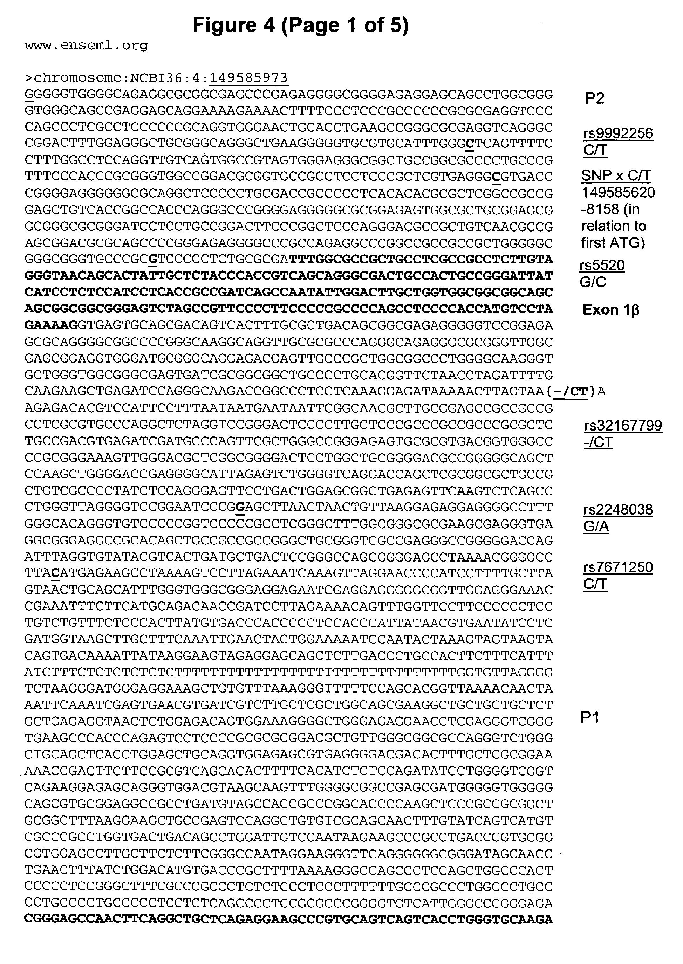

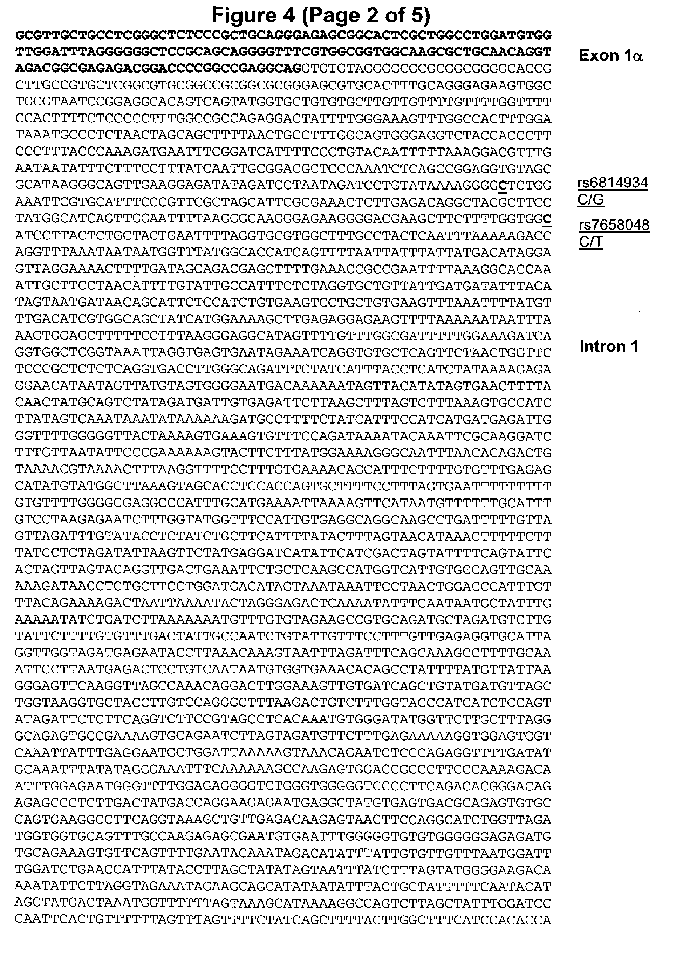

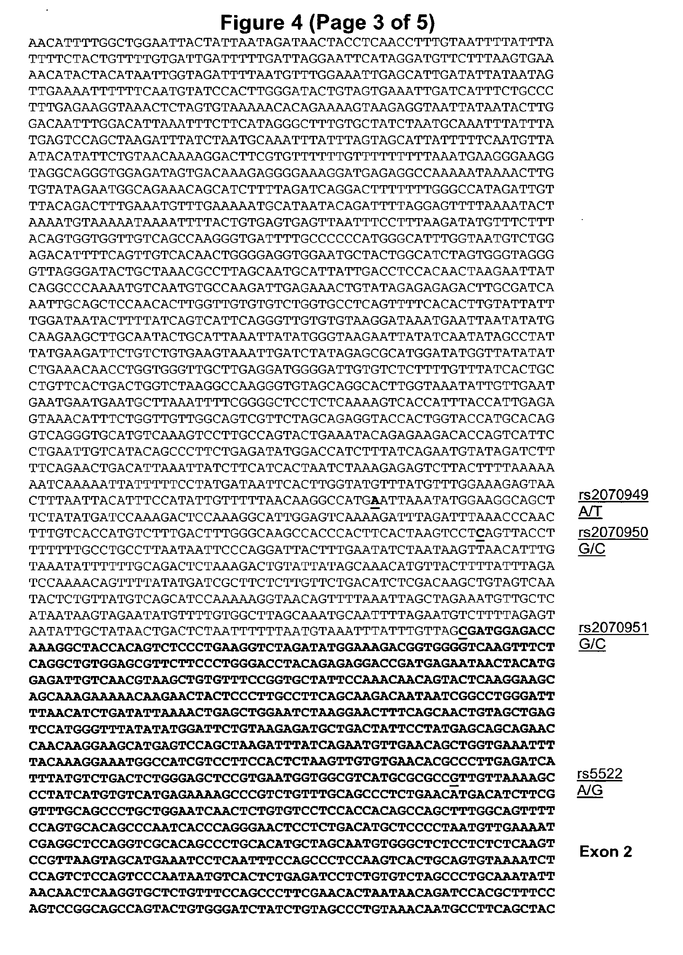

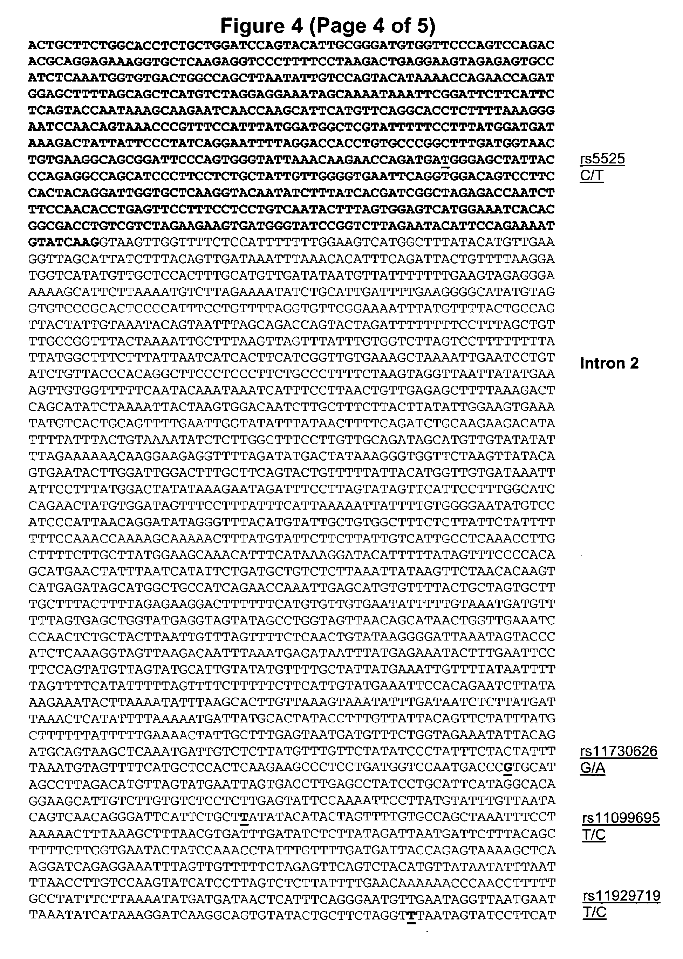

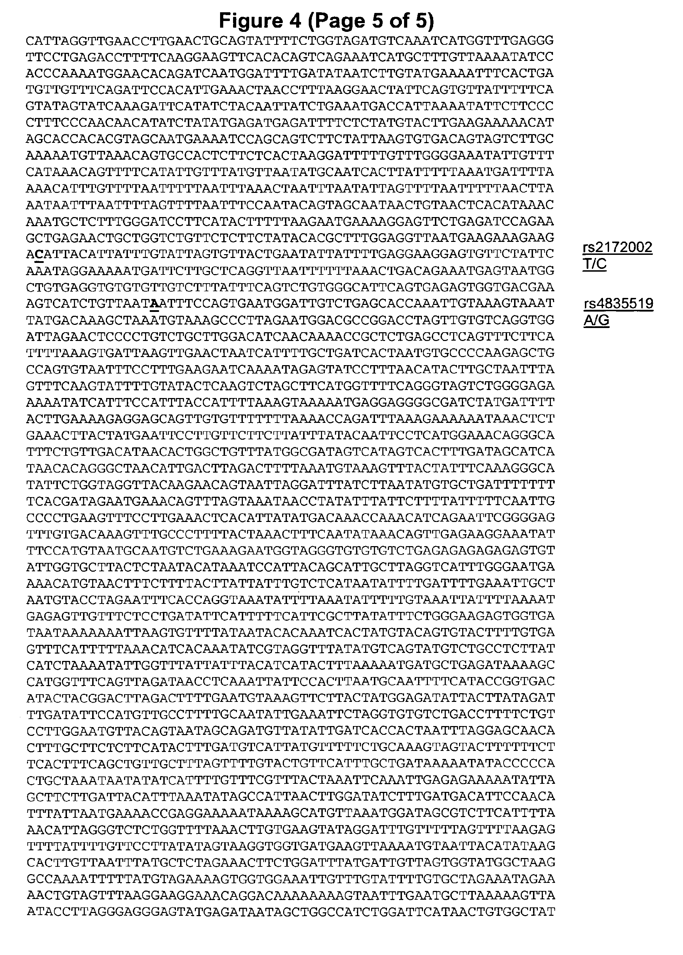

The SNPs rs3216799, rs6814934, rs7658048, rs2070950 and rs2070951 reside within the human mineralocorticoid receptor (MR) gene. The human MR gene is disclosed in GenBank Accession No NC_000004.11 and the sequence of a particular variant of the gene, the position of various SNPs including rs3216799, rs6814934, rs7658048, rs2070950 and rs2070951, and their possible alleles, are given in Figure 4.

Preferably, one or more of rs3216799, rs6814934, rs7658048, rs2070950 and rs2070951 are genotyped, for example, two or more, three or more, four or more, or all five of rs3216799, rs6814934, rs7658048, rs2070950 and rs2070951 are genotyped.

In addition to rs3216799, rs6814934, rs7658048, rs2070950 and rs2070951 which the inventors have shown are individually associated with dispositional optimism, it will be appreciated that polymorphic sites in linkage disequilibrium with one or more of these SNPs are also useful in assessing the susceptibility of a subject to an anxiety disorder or depression. Thus, the Invention includes genotyping one or more polymorphic sites which are in linkage disequilibrium with any one or more of rs3216799, rs6814934, rs7658048, rs2070950 and rs2070951, either instead of or in addition to genotyping one or more of rs3216799, rs6814934, rs7658048, rs2070950 and rs2070951. By 'polymorphic sites in linkage disequilibrium' we include one or more base pairs or other structural features of the nucleic acid (such as an insertion or deletion or repeat sequence) that are in linkage disequilibrium with any one or more of rs3216799, rs6814934, rs7658048, rs2070950 and rs2070951. Typically, the polymorphic sites are SNPs; however, they may be an insertion, a deletion, a microsatellite or an inversion or a combination of these. It is appreciated that the polymorphic sites disclosed herein may or may not be causative. Polymorphic sites which are not causative but which are in linkage disequilibrium with any one of more of rs3216799, rs6814934, rs7658048, rs2070950 and rs2070951 may be used as proxy markers.

In one embodiment, the one or more polymorphic sites in linkage disequilibrium with rs3216799, rs6814934, rs7658048, rs2070950 and rs2070951 are polymorphic sites within the MR gene itself or within the vicinity of the MR gene and form part of the promoter or regulatory architecture. Thus, it will be appreciated that the one or more polymorphic sites may correspond to polymorphic sites within the nucleotide sequence provided in Figure 4.

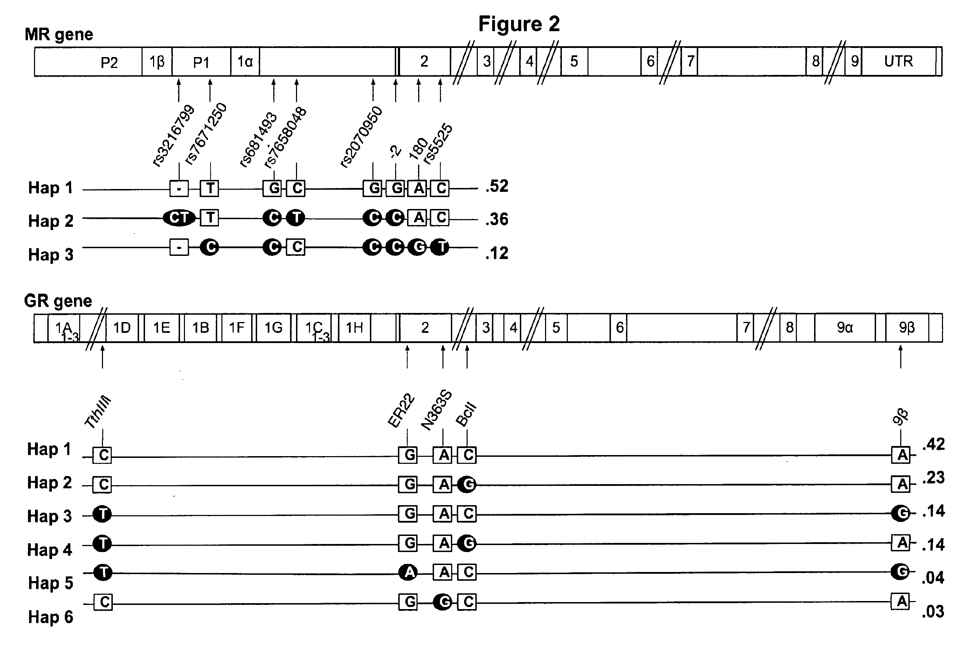

For example, the inventors have identified a haplotype comprising eight SNPs within the MR gene that is associated with dispositional optimism and reduced symptoms of depression (see haplotype 2 of MR gene in Figure 2). The haplotype includes rs3216799, rs6814934, rs7658048, rs2070950 and rs2070951, as well as rs5522, rs5525 and rs7671250. Thus in a particular embodiment, the one or more polymorphic sites in linkage disequilibrium with rs3216799, rs6814934, rs7658048, rs2070950 and rs2070951 may be selected from the group consisting of rs5522, rs5525 and rs7671250 present on the same haplotype. Preferably one or more of rs3216799, rs6814934, rs7658048, rs2070950 and rs2070951 are genotyped, and one or more of rs5522, rs5525 and rs7671250 are genotyped, for example two or more, or all three of rs5522, rs5525 and rs7671250 may be genotyped. In an embodiment, all of rs3216799, rs6814934, rs7658048, rs2070950, rs2070951, rs5522, rs5525 and rs7671250 are genotyped. As seen in Example 1, the SNPs rs3216759, rs6814934, rs7658048, rs2070950, rs2070951, rs5522, rs5525 and rs7671250 with respective alleles '+CT', 'C', 'T', 'C'. 'C', 'A', 'C' and 'T' reside together on haplotype 2 in the MR gene. Therefore, in an embodiment each of rs3216799, rs6814934, rs7658048, rs2070950, rs2070951, rs5522, rs5525 and rs7679250 are genotyped and reduced susceptibility is indicated when their respective alleles are '+CT', 'C', 'T', 'C', 'C', 'A', 'C' and 'T'.

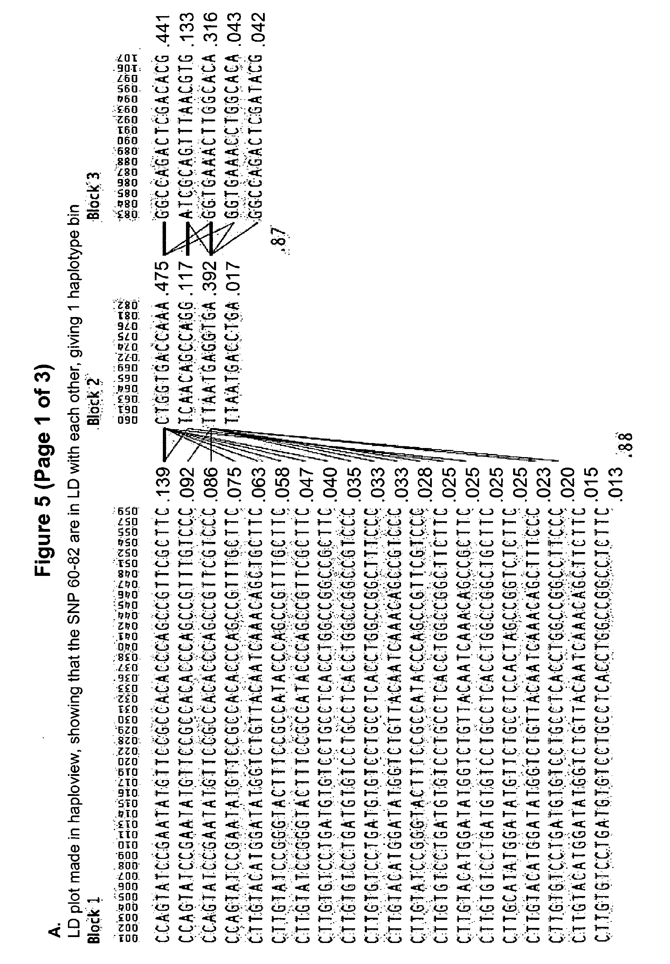

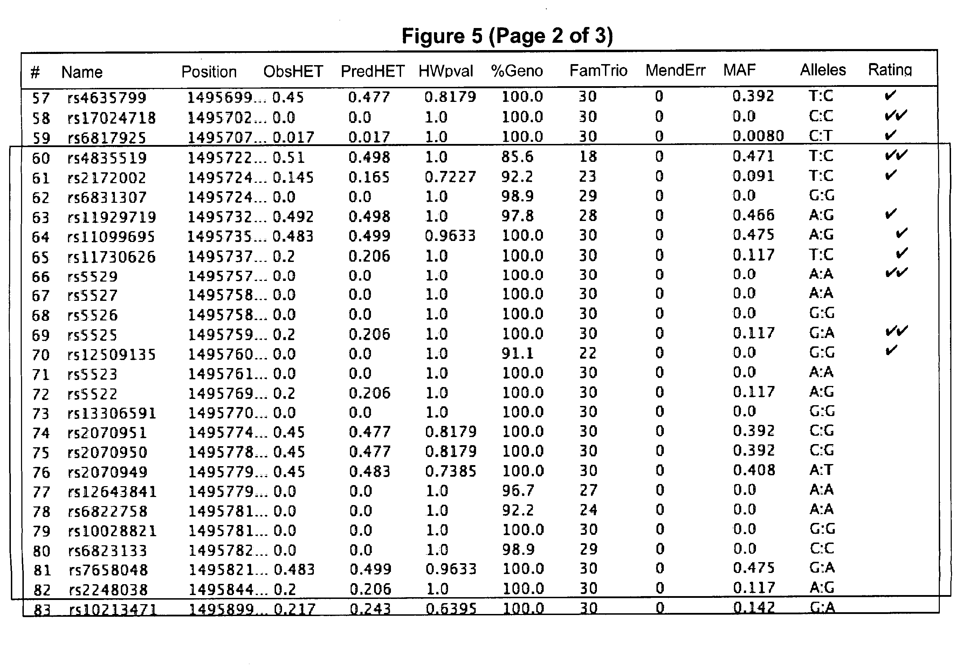

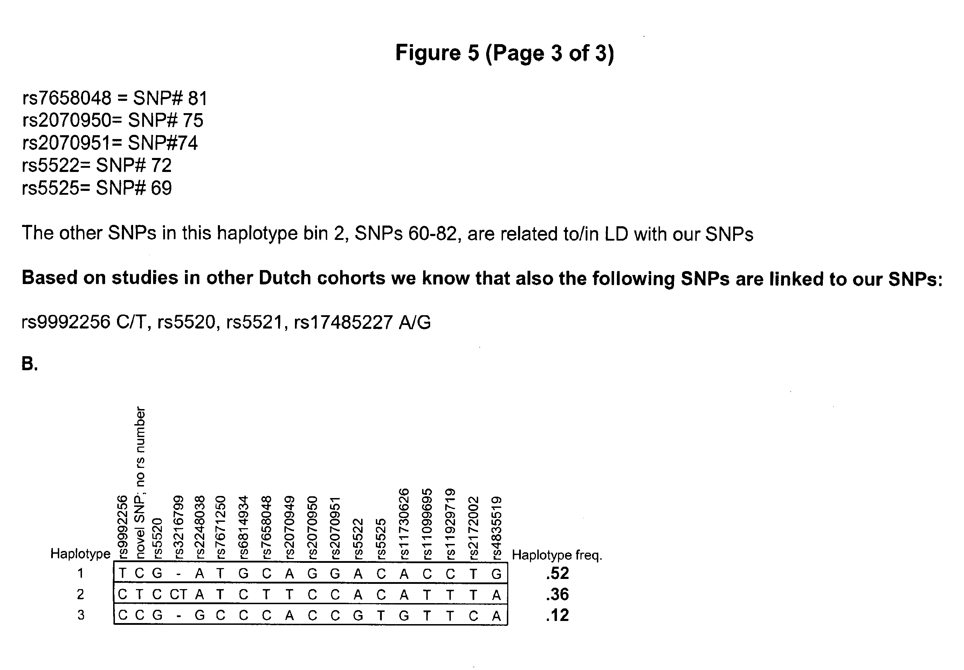

As illustrated in Figure 5, the inventors have identified a further haplotype (haplotype 2A) that comprises the eight SNP alleles in haplotype 2 in addition to a further 10 SNPs. Haplotype 2A comprises rs3216799, rs6814934, rs7658048, rs2070950 and rs2070951, as well as rs5522, rs5525, rs7671250, rs4835519, rs2172002, rs11929719, rs11099695, rs11730828, rs2070949, rs9992256, rsSS20, rs2248038 and SNP x at position 149585620 in the MR gene as numbered in Figure 4. Thus in a particular embodiment, the one or more polymorphic sites in linkage disequilibrium with rs3216799, rs6814934, rs7658048, rs2070950 and rs2070951 may be selected from the group consisting of rs5522, rs5525, rs7671250, rs4835519, rs2172002, rs11929719, rs11099695, rs11730626, rs2070949, rs9992256, rs5520, rs2248038 and SNP x at position 149585620 in the MR gene as numbered in Figure 4 present on the same haplotype. Preferably one or more of rs3216799, rs6814934, rs7658048, rs2070950 and rs2070951 are genotyped, and one or more of (eg at least 2, 3, 4, 5, 6, 7, 8, 9, 10, 11 or 12 or more, or all 13 of) rs5522, rs5525, rs7671250, rs4835519, rs2172002, rs11929719, rs11099695, rs11730626, rs2070949, rs9992256, rs5520, rs2248038 and SNP x at position 149585620 in the MR gene as numbered in Figure 4 are genotyped- In an embodiment each of rs3218799, rs8814934, rs7658048, rs2070950, rs2070951, rs5522, rs5525, rs7671250, rs4835519, rs2172002, rs11929719, rs11099695, rs11730626, rs2070949, rs9992256, rs5520, rs2248038 and SNP x at position 149585620 in the MR gene as numbered in Figure 4 are genotyped and reduced susceptibility is indicated when their respective alleles are '+CT', 'C', 'T', 'C', 'C', 'A'. 'C', 'T', 'A', T, 'T', T, 'A', T, 'C', 'C', 'A' and 'T'.

An analysis of the HapMap database (http://hapmap.ncbi.nlm.nih.gov/) by the inventors (see Figure 5) has identified the following polymorphic sites as being in linkage disequilibrium with one or more of rs3218799, rs8814934, rs7658048, rs2070950 and rs2070951: rs4835519, rs2172002. rs11929719, rs11099695, rs11730626 and rs2070949. Further studies in other Dutch cohorts conducted by the inventors have also identified rs2248038, rs9992256, r55520, and SNP x at position 149585620 in the MR gene as numbered in Figure 4 as being linked to one or more of rs3216799, rs6814934, rs7658048, rs2070950 and rs2070951.

Accordingly, in another embodiment, the one or more polymorphic sites in linkage disequilibrium with any one or more of rs3216799, rs6814934, rs7658048, rs2070950 and rs2070951, may be selected from the group consisting of rs7671250, rs5522, rs5525, rs4835519, rs2172002, rs11929719, rs11099695, rs11730626, rs2070949, rs2248038, rs9992256, rs5520, and SNP x at position 149585620 in the MR gene as numbered in Figure 4. For example, the one or more polymorphic sites may correspond to at least 2, 3, 4, 5, 6, 7, 8, 9, 10, 11, 12 or all, 13, of rs7671250, rs5522, rs5526, rs4835519, rs2172002, rs11929719, rs11099695, rs11730626, rs2070949, rs2248038, rs9992256, rs5520, and SNP x at position 149585620 in the MR gene as numbered in Figure 4. It will be appreciated that any one or more of these polymorphic sites may be genotyped alone or in combination with any one or more of rs3216799, rs6814934, rs7658048, rs2070954 and rs2070951. When any of rs5522, rs5525, rs7671250, rs4835519, rs2172002, rs11929719, rs11099695, rs11730626, rs2070949, rs9992256, rs5520, rs2248038 and SNP x at position 149585620 in the MR gene as numbered in Figure 4 are genotyped, reduced susceptibility is indicated when their respective alleles are 'A', 'C', 'T', 'A', 'T', 'T', 'T', 'A', 'T', 'C', 'C', 'A' and 'T', which are in linkage disequilibrium with the respective '+CT', 'C', 'T', 'C' and 'C' alleles of rs3216799, m6814934, rs7658048, rs2070950 and rs2070951.

It will be appreciated that the one or more polymorphic sites in linkage disequilibrium with one or more of rs3216799, rs6814934, rs7658048, rs2070950 and rs2070951, may be within a genomic region encompassing the MR gene, rather than, or in addition to, being within the MR gene itself. Thus, in humans, where the MR gene resides on chromosome 4, the one or more polymorphic sites may be a site anywhere on chromosome 4 that is in linkage disequilibrium with one or more of rs3216799, rs6814934, rs7658048, rs2070950 and rs2070951. For example, the one or more polymorphic sites may be within 500 kb or within 100 kb or within 50 kb, or within 40kb, or within 30 kb, or within 20 kb, or within 10 kb, or within 5 kb of the MR gene. This may be measured from the 5' end of the first exon of the gene going in the 5' direction and from the 3' end of the last exon in the gene going in the 3' direction. Variation may be found within the exons or within the introns or In regulatory regions of the genes such as the promoter region.

Further polymorphic sites that are in linkage disequilibrium with one or more of rs3216799, rs6814934, rs7658048, rs2070950 and rs2070951, or any other SNP, may be determined, for example, by using relevant data from linkage disequilibrium maps of the human genome (when the subject is human) which have been created using HapMap data (see Tapper et al (2005) Proc. Natl. Acad. Sci, USA 102, 11835-11839 for methodology).

The inventors have associated each of the respective '+CT', 'C', 'T', 'C' and 'C' alleles of rs3216799, rs6814934, rs7658048, rs2070950 and rs2070951 with dispositional optimism. Thus it will be appreciated that reduced susceptibility to an anxiety disorder or depression Is indicated when the alleles of the one or more SNPs are identified as being respectively one or more of '+CT', 'C', **'T',** 'C' and 'C'. Similarly, when genotyping one or more polymorphic sites in linkage disequilibrium with one or more of rs3216799, rs6814934, rs7658048, rs2070950 and rs2070951, it will be appreciated that reduced susceptibility to an anxiety disorder or depression is indicated when the alleles of the polymorphic sites are those which are in linkage disequilibrium with the '+CT', 'C', 'T', 'C' and 'C' alleles of respective SNPs rs3216799, rs6814934, rs7658048, rs2070950 and rs2070951. For example, when genotyping one or more of rs5522, rs5525 and rs7671250, reduced susceptibility is indicated when the allele is found to be respectively 'A', 'C' and T', which are in linkage disequilibrium with the '+CT'. 'C', 'T', 'C' and 'C' alleles of respective SNPs rs3216799, rs6814934, rs7658048, rs2070950 and rs2070951, and which together form a haplotype. Similarly, when genotyping one or more of rs4835519, rs2172002, rs11929719, rs11099695, rs11730626, rs2070949, rs9992258, rs5520, rs2248038 and SNP x at position 149585620 in the MR gene as numbered in Figure 4, reduced susceptibility is indicated when their respective alleles are 'A', 'T', 'T', 'T', 'A', T, 'C', 'C', 'A' and T, which are in linkage disequilibrium with the respective '+CT', 'C', 'T', 'C' and 'C' alleles of rs3216799, rs6814934, rs7658048, rs2070950 and rs2070951.

Although the inventors have found that rs3216799, rs6814934, rs7658048, rs2070950 and rs2070951 with respective alleles '+CT', 'C', 'T', 'C' and 'C' are individually associated with dispositional optimism, it is appreciated that SNP alleles generally occur in combination as haplotypes. Thus, in a particularly preferred embodiment, genotyping any one or more single nucleotide polymorphisms (SNPs) selected from the group consisting of rs3216799, rs6814934, rs7658048, rs2070950 and rs2070951, and/or one or more polymorphic sites which are in linkage disequilibrium with any one or more SNPs selected from the group consisting of rs3216799, rs6814934, rs7658048, rs2070950 and rs2070951, is used to determine whether the subject has a haplotype comprising rs3216799, rs6814934, rs7658048, rs2070950 and rs2070951 with respective alleles '+CT', 'C', 'T', 'C' and 'C'.

Accordingly, the invention provides a method of assessing the susceptibility of a subject to an anxiety disorder or depression, the method comprising determining whether the subject has a haplotype comprising rs3216799, rs6814934, rs7658048, rs2070950 and rs2070951 with respective alleles '+CT', 'C', 'T', 'C' and 'C', or a haplotype that is genetically equivalent thereto.

As discussed above and in Examples 1 and 2, the inventors have investigated associations between variants of the MR gene and each of dispositional optimism and depression. Haplotype reconstruction identified three main haplotypes shown in Figure 2 (haplotypes 1, 2, and 3) with different allelic combinations of the same SNPs.

By 'haplotype 1' we include the meaning of a haplotype comprising SNPs rs3296799, rs6814934, rs7668048, rs2070950, rs2070951, rs5522, rs5525 and rs7671250 with respective alleles '-CT', 'G', 'C', 'G', 'G', 'A', 'C' and 'T'.

By 'haplotype 2' we include the meaning of a haplotype comprising SNPs rs3216799, rs6814934, rs7658048, rs2070950, rs2070951, rs5522, rs5525 and rs7671250 with respective alleles '+CT', 'C', T, 'C', 'C', 'A', 'C' and T.

By 'haplotype 3' we include the meaning of a haplotype comprising SNPs rs3216799, rs6814934, rs7658048, rs2070950, rs2070951, rs5522, rs5525 and rs7671250 with respective alleles '-CT', 'C', 'C', 'C', 'C', 'G', 'T' and 'C'.

Haplotype 2 comprises rs3216799, rs6814934, rs7658048, ras2070950 and rs2070951 with respective alleles '+CT', 'C', T, 'C' and 'C', and the inventors have associated this haplotype with both dispositional optimism and reduced symptoms of depression. Thus, in a preferred embodiment, the haplotype comprising rs3216799, rs6814934, rs7658048, rs2070950 and rs2070951 with respective alleles '+CT', 'C', 'T', 'C' and 'C', is 'haplotype 2' comprising rs3216799, rs6814934, rs7658048, rs2070950, rs2070951, rs5522, rs5525 and rs7671250 with respective alleles '+CT', 'C, 'T', 'C', 'C', 'A', 'C' and 'T'. It will be appreciated that the presence of haplotype 2 in the DNA of a subject may be used as an indicator that the subject has reduced susceptibility to an anxiety disorder or depression.

It will be understood that one or more further polymorphic sites may be in linkage disequilibrium with the alleles of the SNPs in haplotypes 1, 2 or 3, and so one or more further polymorphic sites (eg SNPs) may reside on the same haplotype. For example, Figure 5B shows that each of haplotypes 1, 2 and 3 defined above are part of haplotypes comprising further SNPs (giving rise to haplotypes 1A, 2A and 3A respectively).

By 'haplotype 1A', we include the meaning of a haplotype comprising SNPs rs9992256, SNP x at position 149585620 in the MR gene as numbered in Figure 4, rs5520, rs3216799, rs2248038, rs7671250, rs6814934, rs7658048, rs2070949, rs2070950, rs2070951, rs5522, rs5525, rs17730526, rs11099695, rs11929719, rs2172002 and rs4835519 with respective alleles 'T', 'C', 'G', '-', 'A', 'T', 'G', 'C', 'A', 'G', 'G', 'A', 'C', 'A', 'C', 'C', 'T' and 'G' (see Figure 5B).

By 'haplotype 2A', we include the meaning of a haplotype comprising SNPs rs9992256, SNP x at position 149585620 in the MR gene as numbered in Figure 4, rs5520, rs3216799, rs2248038, rs7671250, rs6814934, rs7658048, rs2070949, rs2070950, rs2070951, rs5522, rs5525, rs17730626, rs11099695, rs11929719, rs2172002 and rs4835519 with respective alleles 'C. 'T', 'C', '+CT', 'A', 'T', 'C', 'T', 'T', 'C', 'C', 'A', 'C', 'A', 'T', 'T', 'T' and 'A' (see Figure 5B).

By 'haplotype 3A' we include the meaning of a haplotype comprising SNPs rs9992256, SNP x at position 149585620 in the MR gene as numbered in Figure 4, rs5520, rs3216799, rs2248038, rs7671250, rs6814934, rs7658048, rs2070949, rs2070950, rs2070951, rs5522, rs5525, rs17730626, rs11099695, rs11929719, rs2172002 and rs4835519 with respective alleles 'C', 'C', 'G', '-', 'G', 'C', 'C', 'C', 'A', 'C', 'C', 'G', T, 'G', T, T, 'C' and 'A' (see Figure 5B).

Haplotype 2A comprises rs3216799, rs6814934, rs7658048, rs2070950 and rs2070951 with respective alleles '+CT', 'C', 'T', 'C' and 'C', and so in a preferred embodiment, the haplotype comprising rs3216799, rs6814934, rs7658048, rs2070950 and rs2070951 with respective alleles '+CT', 'C', 'T', 'C' and 'C', is 'haplotype 2A' comprising SNPs rs9992256, SNP x at position 149585620 In the MR gene as numbered in Figure 4, rs5520, rs3216799, rs2248038, rs7671250, rs6814934, rs7658048, rs2070949, rs2070950, rs2070951, rs5522, rs5525, rs17730626, rs11099695, rs11929719, rs2172002 and rs4835519 with respective alleles 'C', 'T', 'C', 'CT', 'A', 'T', 'C', T, 'T', 'C', 'C', 'A', 'C', 'A', 'T', 'T', 'T' and 'A'. It will be appreciated that the presence of haplotype 2A in the DNA of a subject may be used as an indicator that the subject has reduced susceptibility to an anxiety disorder or depression.

Since the inventors' haplotype reconstruction of the MR gene variants identified only three main haplotypes (eg haplotypes 1-3 or haplotypes 1A-3A), it is appreciated that the presence of 'haplotype 2' or 'haplotype 2A' may be determined by genotyping only two of the SNPs within the haplotype, for example, so as to distinguish 'haplotype 2' or 'haplotype 2A', from 'haplotype 1' or 'haplotype 1A', or from 'haplotype 3' or 'haplotype 3A'. For example, genotyping rs6814934 and rs7658048, or genotyping rs2070951 and rs5522, or genotyping rs9992256 and rs5520 may be used to distinguish between the presence of 'haplotype 2A' as opposed to 'haplotype 1A' or haplotype 3A'. Taking the combination of rs2070951 and rs5522 as a particular example; if a chromosome contains haplotype 1A then the genotype results will be G and A respectively; if the chromosome contains haplotype 2A then the genotype results will be C and A respectively; and if the chromosome contains haplotype 3A then the genotype results will be C and G respectively. Any particular combination of two SNPs may be selected for genotyping in order to distinguish between haplolypes 1-3 by reference to Figure 2, or to distinguish between haplotypes 1A-3A by reference to Figure 5B. However, it is appreciated that it may be desirable to genotype more than two SNPs.

Thus in one embodiment, the method comprises genotyping two or more of (eg at least 3, 4, 5, 6, 7 or all 8 of) rs3216799, rs6814934, rs7658048, rs2070950, rs2070951, rs5522, rs5525 and rs7671250. In this way, at least two SNPs may be genotyped in order to distinguish between each of haplotypes 1-3.

In another embodiment, the method comprises genotyping two or more of (eg at least 3, 4, 5, 6, 7, 8, 9, 10, 11, 12, 13, 14, 15, 16, 17 or all 18 of) rs9992256, SNP x at position 149585620 in the MR gene as numbered in Figure 4, rs5520, rs3216799, nS2248038, rs7671250, rs6814934, rs7658048, rs2070949, rs2070950, rs2070951, rs5522, rs5525, rs17730626, rs11099695, rs11929719, rs2172002 and rs4835519. In this way, at least two SNPs may be genotyped in order to distinguish between each of haplotypes 1A-3A.

As well as genotyping the SNPs within a haplotype to determine whether or not that haplotype is present, it is appreciated that one or more polymorphic sites that are in linkage disequilbrium with (and so act as a tag of) that haplotype may be genotyped. Thus, the methods of the invention may involve genotyping one or more polymorphic sites that are in linkage disequilbrium with a haplotype comprising rs3216799, rs6814934, rs7658048, rs2070950 and rs2070951 with respective alleles '+GT', 'C', 'T', 'C' and 'C', such as haplotype 2 or haplotype 2A above, in order to determine whether that particular haplotype is present.

By 'genotyping', we include the meaning of determining the genotype of at least one of the SNPs described herein. In this way, the particular base or allele of a polymorphic site (eg SNP) becomes known. It is appreciated that by 'genotyping' we Include the direct determination of a particular base or allele of a polymorphic site, as well as an indirect indicator of a particular base or allele of a polymorphic site.

It will be appreciated that genotyping any one or more of the polymorphic sites (eg SNPs) described above conveniently comprises contacting a sample of nucleic acid from the subject with one or more nucleic acid molecules that hybridise selectively to a genomic region encompassing anyone or more of the polymorphic sites (eg SNPs).

By 'hybridising selectively to a genomic region encompassing' any 'one or more SNPs' or 'one or more polymorphic sites', we Include the meaning of a nucleic acid molecule hybridising to one allele of a polymorphic site (eg SNP) but not to the other allele of that polymorphic site (eg SNP). Thus, whether or not a given nucleic acid hybridises to a genomic region encompassing a polymorphic site (eg SNP) can be used as an indicator of which allele is present at that site.

It will be appreciated that a given nucleic acid molecule may hybridise selectively to more than one polymorphic site (eg SNP), for example a given nucleic molecule may hybridise selectively to polymorphic sites that are in close proximity to each other.

The sample of nucleic acid from the subject may be any suitable sample and includes genomic DNA, RNA and cDNA. Genomic DNA is preferred because most SNPs are in non-translated regions, but for the avoidance of doubt and where the context permits it, the sample also includes cDNA and mRNA. The sample of nucleic acid may be obtained in any suitable way, for example from a blood sample or from a mouthwash or from a buccal swab or other tissue sample. The sample of nucleic acid which is analysed may be a sample obtained from the subject. However, typically, the sample of nucleic acid which is analysed is one which has been amplified from the Immediate sample obtained from the subject. For example, polymerase chain reaction (PCR), or other *in vitro* amplification techniques such as the ligase chain reaction (LCR), may conveniently be used to amplify the sample. Thus it will be appreciated that the sample of nucleic acid from the subject may be subjected to a nucleic acid amplification before contacting with one or more nucleic acid molecules that hybridise selectively to any one or more of the SNPs described above, such as those selected from the group consisting of rs3216799, rs6814934, rs7858048, rs2070950 and rs2070951, and/or to one or more polymorphic sites which are in linkage disequilibrium with any one or more SNPs selected from the group consisting of rs3216799, rs6814934, rs7658048, rs2070950 and rs2070951.

As is explained in more detail below, the one or more nucleic acid molecules that hybridise selectively to the a genomic region encompassing any one or more of the SNPs described above, such as those selected from the group consisting of rs3216799, rs6814934, rs7658048, rs2070950 and rs2070951, and/or a genomic region encompassing one or more polymorphic sites which are in linkage disequilibrium with any one or more SNPs selected from the group consisting of rs3216799, rs6814934, rs7658048, rs2070950 and rs2070951, may be, for example, a PCR primer which is used to amplify a genomic region containing a polymorphic site (eg an SNP), or may be a nucleic acid which is able to hybridise at or dose to a polymorphic site (eg an SNP) and be used to determine the nucleic acid sequence variant(s) at the polymorphic site. When the subject is a human, it will be appreciated that the genomic region corresponds to the nucleic acid of chromosome 4.

By "selectively hybridising" we include the meaning that the nucleic acid molecule has sufficient nucleotide sequence similarity with the said genomic DNA or cDNA or mRNA that it can hybridise under highly stringent conditions. As is well known in the art, the stringency of nucleic acid hybridisation depends on factors such as length of nucleic acid over which hybridisation occurs, degree of identity of the hybridising sequences and on factors such as temperature, ionic strength and CG or AT content of the sequence. Thus, any nucleic acid which is capable of selectively hybridising as said is useful in the practice of the invention. It Is preferred that the nucleic acid which selectively hybridises, selectively hybridises to the MR gene, preferably the human MR gene.

An example of a typical hybridisation solution when a nucleic acid is immobilised on a nylon membrane and the probe nucleic acid is ≥ 500 bases or base pairs is:
6 x SSC (saline sodium citrate)
0.5% sodium dodecyl sulphate (SDS)
100 µg/ml denatured, fragmented salmon sperm DNA

The hybridisation is performed at 68°C. The nylon membrane, with the nucleic acid immobilised, may be washed at 68°C in 1 x SSC or, for high stringency, 0.1 x SSC.

20 x SSC may be prepared in the following way. Dissolve 175.3 g of NaCl and 88.2 g of sodium citrate in 800 ml of H₂O. Adjust the pH to 7.0 with a few drops of a 10 N solution of NaOH. Adjust the volume to 1 litre with H₂O. Dispense into aliquots. Sterilize by autoclaving.

An example of a typical hybridisation solution when a nucleic acid is immobilised on a nylon membrane and the probe is an oligonucleotide of between 15 and 50 bases is:
3.0 M trimethylammonium chloride (TMACl)
0.01 M sodium phosphate (pH 6.8)
1 mm EDTA (pH 7.6)
0.5% SDS
100 µg/ml denatured, fragmented salmon sperm DNA
0.1% nonfat dried milk

The optimal temperature for hybridisation is usually chosen to be 5°C below the T₁ for the given chain length. T_{I} is the irreversible melting temperature of the hybrid formed between the probe and its target sequence. Jacobs et al (1988) Nucl. Acids Res. 16, 4637 discusses the determination of Tᵢs. The recommended hybridization temperature for 17-mers in 3 M TMACl is 48-60°C; for 19-mers, it is 55-57°C; and for 20-mers, it is 58-66°C.

Nucleic acids which can selectively hybridise to the said DNA (such as human DNA) include nucleic acids which have >95% sequence identity, preferably those with >98%, more preferably those with >99% sequence identity, for example 100% sequence identity, over at least a portion of the nucleic acid with the said DNA or cDNA. As is well known, mammalian (such as human) genes usually contain introns such that, for example, a mRNA or cDNA derived from a gene within the said human DNA would not match perfectly along its entire length with the said human DNA but would nevertheless be a nucleic acid capable of selectively hybridising to the said human DNA. Thus, the invention specifically Includes nucleic acids which selectively hybridise to a cDNA but may not hybridise to an MR gene, or *vice* versa. For example, nucleic acids which span the intron-exon boundaries of the MR gene may not be able to selectively hybridise to the MR cDNA respectively.

"Nucleic acid which selectively hybridises* is typically nucleic acid which will amplify DNA from the said region of DNA by any of the well known amplification systems such as those described in more detail below, in particular the polymerase chain reaction (PCR). Suitable conditions for PCR amplification include amplification in a suitable 1 x amplification buffer:
10 x amplification buffer is 500 mM KCI; 100 mM Tris.Cl (pH 8.3 at room temperature); 15 mM MgCi₂: 0.1% gelatin.

A suitable denaturing agent or procedure (such as heating to 95°C) is used in order to separate the strands of double-stranded DNA.

Suitably, the annealing part of the amplification is between 37°C and 60°C, preferably 50°C.

Various methods are known in the art for genotyping polymorphic sites, including SNPs, in the method of the invention.

For example, methods of determining polymorphic sites within a nucleic acid may involve sequencing of DNA at one or more of the relevant positions within the relevant region, including direct sequencing; direct sequencing of PCR-amplified products; differential hybridisation of an oligonucleotide probe designed to hybridise at the relevant positions within the relevant region (conveniently this uses immobilised oligonucleotide probes in, so-called, "chip" systems which are well known in the art); denaturing gel electrophoresis following digestion with an appropriate restriction enzyme, preferably following amplification of the relevant DNA regions; S1 nuclease sequence analysis; non-denaturing gel electrophoresis, preferably following amplification of the relevant DNA regions; conventional RFLP (restriction fragment length polymorphism) assays; heteroduplex analysis; selective DNA amplification using oligonucleotides; fluorescent *in-situ* hybridisation (FISH) of interphase chromosomes; ARMS-PCR amplification Refractory Mutation System-PCR) for specific mutations; cleavage at mismatch sites in hybridised nucleic acids (the cleavage being chemical or enzymatic); SSCP single strand conformational polymorphism or DGGE (discontinuous or denaturing gradient gel electrophoresis); analysis to detect mismatch in annealed normal/mutant PCR-amplified DNA; and protein truncation assay (translation and transcription of exons - if a mutation introduces a stop codon a truncated protein product will result). Other methods may be employed such as detecting changes in the secondary structure of single-stranded DNA resulting from changes in the primary sequence, for example, using the cleavase I enzyme. This system is commercially available from GibcoBRL, Life Technologies, 3 Fountain Drive, Inchinnan Business Park, Paisley PA4 9RF, Scotland. SNP changes may also be detected by DNA high resolution melt assays or by the Taqman assay system (see Heid et al (1996) Genome Res. 6, 986-994).

It will be appreciated that the methods of the invention may also be carried out on "DNA chips". Such "chips" are described in US 5,445,934 (Affymetrix; probe arrays), WO 96/31622 (Oxford; probe array plus ligase or polymerase extension), and WO 95/22058 (Affymax; fluorescently marked targets bind to oligomer substrate, and location in array detected); all of these are Incorporated herein by reference.

Detailed methods of mutation detection are described in "Laboratory Protocols for Mutation Detection" 1996, ed. Landegren, Oxford University Press on behalf of HUGO (Human Genome Organisation).

It is preferred if RFLP is used for the detection of fairly large (≥ 500bp) deletions or insertions which may be in linkage disequilibrium with any one or more of rs3216799, rs6814934, rs7658048, rs2070950 and rs2070951. Southern blots may be used for this embodiment of the invention.

PCR amplification of smaller regions (for example up to 300bp) to detect small changes greater than 3-4 bp insertions or deletions may be preferred. Amplified sequence may be analysed on a sequencing gel, and small changes (minimum size 3-4 bp) can be visualised. Suitable primers are designed as herein described.

In addition, using either Southern blot analysis or PCR, restriction enzyme variant sites may be detected. For example, for analysing variant sites In genomic DNA restriction enzyme digestion, gel electrophoresis, Southern blotting, and hybridisation specific probe (for example any suitable fragment derived from the MR cDNA or gene) may be used. For example, for analysing variant sites using PCR DNA amplification, restriction enzyme digestion, gel detection by ethidium bromide, silver staining or incorporation of radionucleotide or fluorescent primer in the PCR may be used.

Other suitable methods include the development of allele specific oligonucleotides (ASOs) for specific mutational events.

Primers which are suitable for use in a polymerase chain reaction (PCR; Saiki et al (1988) Science 239, 487-491) are preferred.

Any of the nucleic acid amplification protocols can be used in the method of the invention including the polymerase chain reaction, QB replicase and ligase chain reaction. Also, NASBA (nucleic acid sequence based amplification), also called 3SR, can be used as described in Compton (1991) Nature 350, 91-92 and AIDS (1993), Vol 7 (Suppl 2), S108 or SDA (strand displacement amplification) can be used as described in Walker et al (1992) Nucl. Acids Res. 20, 1691-1696. The polymerase chain reaction is particularly preferred because of its simplicity.

The methods of the invention may make use of a difference in restriction enzyme cleavage sites caused by mutation. A non-denaturing gel may be used to detect differing lengths of fragments resulting from digestion with an appropriate restriction enzyme.

An "appropriate restriction enzyme" is one which will recognise and cut one polymorphic sequence and not another polymorphic sequence or *vice versa*. The sequence which is recognised and cut by the restriction enzyme (or not, as the case may be) can be present as a consequence of the mutation or it can be introduced into the normal or mutant allele using mismatched oligonucleotides in the PCR reaction. It Is convenient if the enzyme cuts DNA only infrequently, in other words if it recognises a sequence which occurs only rarely.

In another method, a pair of PCR primers are used which match (i.e. hybridise to) either one polymorphic site or the other polymorphic site but not both. Whether amplified DNA is produced will then indicate whether one or the other allele is present.

Any of the above methods may be employed in the method of the invention. In a particularly preferred embodiment, the genotyping may be carried out using a Sequenom Mass ARRAY iPLEX assay (Sequenom, San Diego, CA, USA) or the like, as described In Example 1. In this assay, after amplification by PCR, a primer extension is used to introduce allele specific mass differences for a given SNP which can be detected using mass spectrometry.

Typically, the subject is a human subject, and preferably a female human subject. In this case, the one or more polymorphic sites in linkage disequilibrium with one or more of rs3216799, rs6814934, rs7658048, rs2070950 and rs2070951, or with any of the SNPs in haplotype 2 or haplotype 2A, would be within the human MR gene or in a genomic region encompassing the human MR gene (ie chromosome 4).

The method of the invention may comprise analysing a further genetic locus of the subject associated with an anxiety disorder or depression. Other genetic loci which have been associated with an anxiety disorder or depression in humans include the glucocorticoid receptor (GR) gene (eg any one or more of rs6195, rs6196, rs6189, rs6190, rs41423247, rs6198, rs10052957, rs10482605, rs1866388, rs2918419 and rs860458, may be genotyped - see DeRIJk NIM 16, pp 340-352, 2009), a heat shock protein gene such as FKBP 5 (eg any one or more of rs9296158, rs3800373, rs1360780 and rs9470080 may be genotyped - see Binder JAMA 299, pp1291-1305, 2008), the P-glycoprotein (P-gp) gene (eg any one or more of rs2032583 and rs2235015 may be genotyped - see Uhr et al Neuron 57, pp203-209, 2008), the Corticotropin Releasing hormone Receptor 1 gene (CRHR1; eg rs878886 may be genotyped) or the Vasopressin 1B Receptor gene (AVPR1B; eg r528632197 may be genotyped, see Keck et al, AJ Med Gen Part B, NeuroPsychi Res, 147B(7):1196-204, 2008). Thus, an analysis at any one or more of these loci may be carried out in addition to the analysis of the one or more polymorphic sites (eg SNPs) described above, such as those selected from the group consisting of rs3216799, rs6814934, rs7658048, rs2070950 and rs2070951, and/or the one or more polymorphic sites which are in linkage disequilibrium with any one or more SNPs selected from the group consisting of rs3216799, rs6814934, rs7658048, rs2070950 and rs2070951.

It will be appreciated that with current technology multiple mutations may be identified in a subject, for example from a single DNA sample. The skilled person may readily use the information contained herein to genotype not only the polymorphic sites (eg SNPs) described above that the inventors have associated with dispositional optimism and anxiety, but also one or more additional genetic loci as mentioned above. As is discussed below, the invention therefore also includes kits of parts and DNA chips which are specifically designed to be useful in assessing a subject's susceptibility to an anxiety disorder or depression.

It will be appreciated that it may be desirable to obtain data on other risk factors for an anxiety disorder or depression, in addition to the genotyping methods described above. Thus, in one embodiment, one or more of the age, sex, body mass index (BMI), smoking status, childhood trauma, or stress status (eg chronic or acute) of the subject is considered.

The data produced from carrying out the methods of the invention may conveniently be recorded on a data carrier. Thus, the invention includes a method of recording data concerning the susceptibility of a subject to an anxiety disorder or depression using any of the methods of the invention and recording the results on a data carrier. Typically, the data are recorded in an electronic form and the data carrier may be a computer, a disk drive, a memory stick, a CD or DVD or floppy disk or the like.

information recorded on the data carrier may include the name, date of birth, age, sex and smoking status of the subject, as well as genotype information obtained using the methods of the invention.

A second aspect of the invention provides a use of one or more nucleic acid molecules that hybridise selectively to a genomic region encompassing any one or more SNPs selected from the group consisting of rs3216799, rs6814934, rs7658048, rs2070950 and rs2070951, and/or to a genomic region encompassing one or more polymorphic sites which are in linkage disequilibrium with any one or more SNPs selected from the group consisting of rs3216799, rs6814934, rs7658048, rs2070950 and rs2070951 for assessing the susceptibility of a subject to an anxiety disorder or depression, wherein reduced susceptibility is indicated when the allele of the one or more SNPs is respectively one or more of '+CT', 'C', 'T', 'C' and 'C', and/or when the allele of the one or more polymorphic sites is one that is in linkage disequilibrium with the respective one or more '+CT, 'C', 'T', 'C' and 'C' alleles of the one or more SNPs.

In an embodiment, the one or more polymorphic sites (eg SNPs) which are in linkage disequilibrium with any one or more SNPs selected form the group consisting of rs3216799, rs6814934, rs7658048, rs2070950 and rs2070951, are SNPs selected from the group consisting of rs5522, rs5525, rs7671250, rs4835519, rs2172002, rs11929719, rs11099695, rs11730628, rs2070949, rs2248038, rs9992256, rs5520, and SNP x at position 149585620 in the MR gene as numbered in Figure 4. Reduced susceptibility is indicated when the respective alleles of rs5522, rs5525, rs7671250, rs4835519, rs2172002, rs11929719, rs11099695, rs11730626, rs2070949, rs9992256, rs5520, rs2248038 and SNP x at position 149585620 in the MR gene as numbered in Figure 4, are 'A', 'C', 'T', 'A', 'T', T, T, 'A', 'T', 'C', 'C', 'A' and 'T'.

In this aspect of the invention and in the third, fourth, fifth and sixth aspects of the invention described below, it will be appreciated that the any one or more nucleic acid molecules may hybridise selectively to one allele of a polymorphic site (eg SNP) but not to the other allele of that polymorphic site (eg SNP). In this way, It can readily be determined which allele of a particular polymorphic site (eg SNP) is present depending upon whether the nucleic acid molecule binds or not

A third aspect of the invention provides one or more nucleic acid molecules that hybridise selectively to a genomic region encompassing any one or more SNPs selected from the group consisting of rs3216799, rs6814934, rs7658048, rs2070950 and rs2070951, and/or to a genomic region encompassing one or more polymorphic; sites which are in linkage disequilibrium with any one or more SNPs selected from the group consisting of rs3216799, rs6814934, rs7658048, rs2070950 and rs2070951 for use in assessing the susceptibility of a subject to an anxiety disorder or depression, wherein reduced susceptibility is indicated when the allele of the one or more SNPs is respectively one or more of '+CT, 'C', 'T', 'C' and 'C', and/or when the allele of the one or more polymorphic sites is one that is in linkage disequilibrium with the respective one or more '+CT', 'C', 'T', 'C' and 'C' alleles of the one or more SNPs.

In an embodiment, the one or more polymorphic sites (eg SNPs) which are in linkage disequilibrium with any one or more SNPs selected form the group consisting of rs3216799, rs6814934, rs7658048, rs2070950 and rs2070951, are SNPs selected from the group consisting of rs5522, rs5525, rs7671250, rs4835519, rs2172002, rs11929719, rs11099695, rs11730626, rs2070949, rs2248038, rs9992256, rs5520, and SNP x at position 149585620 in the MR gene as numbered in Figure 4. Reduced susceptibility is indicated when the respective alleles of rs5522, rs5525, rs7671250, rs4835519, rs2172002, rs11929719, rs11099695, rs11730626, rs2070949, rs9992256, rs5520, rs2248038 and SNP x at position 149585620 in the MR gene as numbered in Figure 4, are 'A'. 'C', 'T', 'A', 'T', 'T', T, 'A', 'T', 'C', 'C', 'A' and 'T'.

A fourth aspect of the Invention provides a use of one or more nucleic acid molecules that hybridise selectively to a genomic region encompassing any one or more SNPs selected from the group consisting of rs3218799, rs6814934, rs7658048, rs2070950 and rs2070951, and/or to a genomic region encompassing one or more polymorphic sites which are in linkage disequilibrium with any one or more SNPs selected from the group consisting of rs3216799, rs6814934, rs7658048, rs2070950 and rs2070951 in the manufacture of a reagent for assessing the susceptibility of a subject to an anxiety disorder or depression, wherein reduced susceptibility is indicated when the allele of the one or more SNPs is respectively one or more of '+CT', 'C', 'T', 'C' and 'C', and/or when the allele of the one or more polymorphic sites is one that is in linkage disequilibrium with the respective one or more '+CT', 'C', 'T', 'C' and 'C' alleles of the one or more SNPs.

In an embodiment, the one or more polymorphic sites (eg SNPs) which are in linkage disequilibrium with any one or more SNPs selected form the group consisting of rs3216799, rs6814934, rs7658048, rs2070850 and rs2070951, are SNPs selected from the group consisting of rs5522, rs5525, rs7671250, rs4835519, rs2172002, rs11929719, rs11099695, rs11730626, rs2070949, rs2248038, rs9992256, rs5520, and SNP x at position 149585620 in the MR gene as numbered in Figure 4. Reduced susceptibility is indicated when the respective alleles of rs5522, rs5525, rs7671250, rs4835519, rs2172002, rs11929719, rs11099695, rs11730626, rs2070949, rs9992256, rs5520, rs2248038 and SNP x are 'A', 'C', 'T'. 'A', 'T', 'T', 'T', 'A', 'T', 'C', 'C', 'A' and T.

A fifth aspect of the invention provides a kit of parts for use in assessing the susceptibility of a subject to an anxiety disorder or depression, the kit comprising one or more nucleic acid molecules that hybridise selectively to a genomic region encompassing any two or more SNPs selected from the group consisting of rs3216799, rs6814934, rs7658048, rs2070950 and rs2070951 (eg with respective alleles '+CT', 'C', 'T', 'C' and 'C'), and/or that hybridise selectively to a genomic region encompassing two or more polymorphic sites in linkage disequilibrium with any one or more SNPs selected from the group consisting of rs3216799, rs6814934, rs7658048, rs2070950 and rs2070951 (eg with respective alleles '+CT', 'C', 'T', 'C' and 'C'). For example, the kit of parts may comprise one or more nucleic acid molecules that hybridise selectively to a genomic region encompassing any two or more SNPs selected from the group consisting of rs3216799, rs6814934, rs7658048, rs2070950 and rs2070951, and/or one or more nucleic acid molecules that hybridise selectively to a genomic region encompassing two or more polymorphic sites in linkage disequilibrium with any one or more SNPs selected from the group consisting of rs3215799, rs6814934, rs7658098, rs2070950 and rs2070951.

Typically, the kit of parts comprises two or more nucleic acid molecules (eg, three or more, or four or more, or five or more nucleic acid molecules) that hybridise selectively to a genomic region encompassing two or more SNPs (eg, three or more, or four or more, or five SNPs) selected from the group consisting of rs3216799, rs6814934, rs7658048, rs2070950) and rs2070951 and/or that hybridise selectively to a genomic region encompassing two or more polymorphic sites (eg three or more, or four or more, or five or more) in linkage disequilibrium with any one or more SNPs selected from the group consisting of rs3216799, rs6814934, rs7658048, rs2070950 and rs2070951.

It is appreciated that the kit of parts contains reagents which are able to be used to determine the genotype of any two or more SNPs (eg three or more, four or more or all five SNPs) selected from the group consisting of rs3216799, rs6814934, rs7658048, rs2070950 and rs2070951, and/or two or more (eg three or more, four or more, or five or more) polymorphic sites in linkage disequilibrium with any one or more SNPs selected from the group consisting of rs3216799, rs6814934, rs7568048, rs2070950 and rs2070951. Thus, the kit of parts may be used to determine whether a subject has a haplotype comprising rs3216799, rs6814934, rs7658048, rs2070950 and rs2070951 with respective alleles '+CT', 'C', 'T', 'C' and 'C', such as haplotype 2 or 2A described above. Conveniently, the kit contains PCR primers which are able to amplify a genomic region encompassing any two or more SNPs (eg three or more, four or more or all five SNPs) selected from the group consisting of rs3216799, rs6814934, rs7658048, rs2070950 and rs2070951, and/or two or more (eg three or more, four or more, or five or more) polymorphic sites in linkage disequilibrium with any one or more SNPs selected from the group consisting of rs3216799, rs6814934, rs7658048, rs2070950 and rs2070951. Conveniently, the kit contains nucleic acid molecules, such as oligonucleotide probes, which can be used to determine the genotype of two or more (eg three or more, four or more or all five SNPs) SNPs selected from the group consisting of rs3216799, rs6814934, rs7658048, rs2070950 and rs2070951 and/or two or more (eg three or more, four or more, or five or more) polymorphic sites in linkage disequilibrium with any one or more SNPs selected from the group consisting of rs3216799, rs6814934, rs7658048, rs2070950 and rs2070951.

In one embodiment, the two or more polymorphic sites in linkage disequilibrium with any one or more SNPs selected from the group consisting of rs3216799, rs6814934, rs7658048, rs2070950 and rs2070951, are two or more of (eg all three of) the SNPs selected from the group consisting of rs5522, rs5525 and rs7671250. Thus, the kit of parts may comprise one or more nucleic acid molecules that selectively hybridise to a genomic region encompassing two or more SNPs selected from the group consisting of rs5522, rs5525 and rs7671250 (eg with respective alleles 'A', 'C' and T).

In another embodiment, the two or more polymorphic sites in linkage disequilibrium with any one or more SNPs selected from the group consisting of rs3216799, rs6814934, rs7658048, rs2070950 and rs2070951, are two or more of (eg at least 2, 3, 4, 5, 6, 7, 8, 9,10,11 or 12, or all of) the SNPs selected from the group consisting of rs5522, rs5525, rs7671250, rs435519, rs2172002, rs11929719, rs11099695, rs11730626, rs2070949, rs2248038, rs9992256, rs5520, and SNP x at position 149585620 in the MR gene as numbered in Figure 4. Thus, the kit of parts may comprise one or more nucleic acid molecules that selectively hybridise to a genomic region encompassing two or more of (eg at least 2, 3, 4, 5, 6, 7, 8, 9, 10, 11 or 12, or all 13, of) the SNPs selected from the group consisting of rs5522, rs5525, rs7671250, rs4835519, rs2172002, rs11929719, rs11099695, rs11730626, rs2070949, rs2248038, rs9992256, rs5520 and SNP x at position 149585620 in the MR gene as numbered In Figure 4. Preferably, the two or more polymorphic sites in linkage disequilibrium with any one or more SNPs selected from the group consisting of rs3216799, rs6814934, rs7658048, rs2070950 and rs2070951, are two or more of rs5522, rs5525, rs7671250, rs4835519, rs2172002, rs11929719, rs11099695, rs11730626, r52070949, rs9992256, rs5520, rs2248038 and SNP x at position 149586620 in the MR gene as numbered in Figure 4, with respective alleles 'A', 'C', `T', 'A', T, T', T, 'A', T', 'C', 'C', 'A' and 'T', which are in linkage disequilibrium with the respective CT, `C', T, 'C' and 'C' alleles of rs3216799. rs6814934, rs7658048, rs2070950 and rs2070951.

In a further embodiment, the kit of parts further comprises or consists of a nucleic acid molecule that hybridises selectively to a further genetic locus associated with an anxiety disorder, such as the GR gene (eg the nucleic acid may hybridise selectively to any of rs6195, rs6198. rs6189. rs6190, rs41423247, rs6198, rs10052957, rs10482605. rs1866388, rs2918419 and rs860458 - see DeRijk NIM 16, pp 340-352, 2009), a heat shock protein gene such as FKBP 5 (eg the nucleic acid may hybridise selectively to any of rs9296158, rs3800373, rs1360780 and rs9470080 - see Binder JAMA 299, pp1291-1305, 2008), the P-glycoprotein (P-gp) gene (eg the nucleic acid may hybridise selectively to rs2032583 or rs2235015 - see Uhr et al Neuron 57, pp203-209, 2008), the Corticotropin Releasing hormone Receptor 1 gene (CRHR1; eg the nucleic acid may hybridises selectively to rs878886) or the Vasopressin 1B Receptor gene (AVPR1B; eg the nucleic acid may hybridise selectively to rs28632197, see Keck et al, AJ Med Gen Part B, NeuroPsychi Res, 147B(7):1196-204, 2008).

The invention also includes a kit of parts for use in assessing the susceptibility of a subject to an anxiety disorder or depression, the kit comprising or consisting of one or more nucleic acid molecules that hybridise selectively to a genomic region encompassing any one or more SNPs selected from the group consisting of rs3216799. rs6814934, rs7658048, rs2070950 and rs2070951 (eg with respective alleles '+CT', 'C', 'T', 'C' and 'C'), and that hybridise selectively to a genomic region encompassing one or more polymorphic sites in linkage disequilibrium with any one or more SNPs selected from the group consisting of rs3216799, rs6814934, rs7658048, rs2070950 and rs2070951 (eg with respective alleles '+CT'. 'C', T, 'C' and 'C'). For example, the kit of parts may comprise one or more nucleic acid molecules that hybridise selectively to a genomic region encompassing any one or more SNPs selected from the group consisting of rs3216799, rs6814934, rs7658048, rs2070950 and rs2070951, and one or more nucleic acid molecules that hybridise selectively to a genomic region encompassing one or more polymorphic sites in linkage disequilibrium with any one or more SNPs selected from the group consisting of rs3216799, rs6814934, rs7658048, rs2070950 and rs2070951. Preferences for the one or more polymorphic sites in linkage disequilibrium with any one or more SNPs selected from the group consisting of rs3216799, rs6814934, rs7658048, rs2070950 and rs2070951 include those described above.

In one embodiment, the kit of parts consists of only the nucleic acid molecules that hybridise as said.

it will be appreciated that the kit of parts of the invention may comprise or consist of at least 2. 3. 4, 5. 6, 7, 8, 9. 10, 11, 12, 13, 14, 15, 16, 17, 18, 19, 20, 21, 22, 23, 24, 26, 30, 35, 40, 45, or 50 different nucleic acid molecules. By different we mean that the nucleic acid molecules have different hybridisation selectivities (eg they may hybridise selectively to different polymorphic sites).

It is also appreciated that the one or more nucleic acid molecules of the kit of parts may hybridise selectively to a region of genome at or close to the given polymorphic sites (eg SNPs).

Typically, the kit of parts of the invention comprises or consists of less than 100 different nucleic acid molecules, eg less than 95, 90, 85, 80, 75, 70, 65, 60, 55, 50, 45, 40, 35, 30, 25, 20, 15 or 10 different nucleic acid molecules.

Typically, the one or more nucleic acid molecules of the kit of parts are less than 100 bases in length, such as less than 90, 80, 70, 60, 50, 40 or 30 bases. For example, the one or more nucleic acid molecules may be between 10 and 30 bases in length, such as 11, 12, 13, 14, 15, 16, 17. 18, 19, 20, 21, 22, 23, 24, 25, 26, 27, 28 or 29 bases in length.

A sixth aspect of the invention provides a solid substrate for use in assessing the susceptibility of a subject to an anxiety disorder or depression, the solid substrate having attached thereto one or more nucleic add molecules that hybridise selectively to a genomic region encompassing any two or more SNPs selected from the group consisting of rs3216799, rs6814934, rs7658048, rs2070050 and rs2070951 (eg with respective alleles '+CT, 'C', T, 'C' and 'C'), and/or that hybridise selectively to a genomic region encompassing two or more polymorphic sites in linkage disequilibrium with any one or more SNPs selected from the group consisting of rs3216799, rs6814934, rs7658048, rs2070950 and rs2070951 (eg with respective alleles '+CT', 'C', 'T', 'C' and `C'). For example, the solid substrate may have attached thereto one or more nucleic acid molecules that hybridise selectively to a genomic region encompassing any two or more SNPs selected from the group consisting of rs3216799, rs6814934, rs7658048, rs2070950 and rs2070951, and/or one or more nucleic acid molecules that hybridise selectively to a genomic region encompassing two or more polymorphic sites in linkage disequilibrium with any one or more SNPs selected from the group consisting of rs3216799, rs6814934, rs7658048, rs2070950 and rs2070951.

Typically, solid substrate has attached thereto two or more nucleic acid molecules (eg, three or more, or four or more, or five or more nucleic acid molecules) that hybridise selectively to a genomic region encompassing two or more SNPs (eg, three or more, or four or more, or five SNPs) selected from the group consisting of rs3216799, rs6814934, rs7658048, rs2070950 and rs2070951 and/or that hybridise selectively to a genomic region encompassing two or more polymorphic sites (eg three or more, or four or more, or five or more) in linkage disequilibrium with any one or more SNPs selected from the group consisting of rs3216799, rs6814934, rs7658048, rs2070950 and rs2070951.

It Is appreciated that the solid substrate may be used to determine whether a subject has a haplotype comprising rs3216799, rs6814934, rs7658048, rs2070950 and rs2070951 with respective alleles '+CT', 'C', T, 'C' and 'C', such as haplotype 2 or 2A described above.

The solid substrate with one or more nucleic acids attached thereto may be a DNA chip or a microarray.

In one embodiment, the solid substrate has only the nucleic acid molecules that hybridise as said attached thereto.

Conveniently, the solid substrate has attached thereto nucleic acid molecules, such as oligonucleotide probes, which can be used to determine the genotype of two or more (eg three or more, four or more or all five SNPs) SNPs selected from the group consisting of rs3216799, rs6814934, rs7658048, rs2070950 and rs2070951 and/or two or more (eg three or more, four or more, or five or more) polymorphic sites in linkage disequilibrium with any one or more SNPs selected from the group consisting of rs3216799, rs6814934, rs7658048, rs2070950 and rs2070951.

In one embodiment, the two or more polymorphic sites in linkage disequilibrium with any one or more SNPs selected from the group consisting of rs3216799, rs6814934, rs7658048, rs2070950 and rs207D951, are two or more of (eg all three of) the SNPs selected from the group consisting of rs5522, rs5525 and rs7671250. Thus, the solid substrate may have attached thereto one or more nucleic acid molecules that selectively hybridise to a genomic region encompassing two or more SNPs selected from the group consisting of rs5522, rs5525 and rs7671250 (eg with respective alleles 'A', 'C' and T).

In another embodiment, the two or more polymorphic sites in linkage disequilibrium with any one or more SNPs selected from the group consisting of rs3296799, rs6814934, rs7658048, rs2070950 and rs20T0951, are two or more of (eg at least 2, 3, 4, 5. 6. 7. 8, 9, 10, 11 or 12. or all 13 of) the SNPs selected from the group consisting of rs5522, rs5525, rs7671250, rs4835519, rs2172002, rs11929719, rs11099695, rs11730626, rs2070949, rs10028821, rs2248038, rs9992256, rs5520, and SNP x at position 149585620 in the MR gene as numbered in Figure 4. Thus, the solid substrate may have attached thereto one or more nucleic acid molecules that selectively hybridise to a genomic region encompassing two or more of (eg at least 2, 3, 4,5,6, 7,8, 9, 10, 11 or 12, or all 13 of) the SNPs selected from the group consisting of rs5522, rs5525, rs7671250, rs4835519, rs2112002, rs11929719, rs11099695, rs11730626, rs2070949, rs2Z48038, rs9992256, rs5520, and SNP x at position 149585620 in the MR gene as numbered in Figure 4- Preferably, the two or more polymorphic sites in linkage disequilibrium with any one or more SNPs selected from the group consisting of rs3216799, rs6814934, rs7658048, rs2070950 and rs2070951. are two or more of rs5522, rs5525, rs7671250, rs4835519, rs2172002, rs11929719, rs11099695, rs11730626. rs2070949, rs9G92256, rs5520. rs2248038 and SNP x at position 149585620 in the MR gene as numbered In Figure 4, with respective alleles 'A', 'C', 'T', 'A', 'T', 'T', 'T', 'A', 'T', 'C', 'C', 'A' and 'T', which are in linkage disequilibrium with the respective CT', 'C', 'T', 'C' and 'C' alleles of r33216799, rs6814934, rs7658048, rs2070950 and rs2070951.

In a further embodiment, the solid substrate has attached thereto a nucleic acid molecule that hybridises selectively to a further genetic locus associated with an anxiety disorder, such as the GR gene (eg the nucleic acid may hybridise selectively to any of rs6195. rs6196, rs6169, rs6190, rs41423247, m6198, rs10052957, rs10482605, rs1866388, rs2918419 and rs860458 - see DeRijk NIM 16, pp 340-352, 2009), a heat shock protein gene such as FKBP 5 (eg the nucleic acid may hybridise selectively to any of rs9296158, rs3800373, rs1360780 and rs9470080 - see Binder JAMA 299, pp1291-9305, 2008), the P-glycoprotein (P-gp) gene (eg the nucleic acid may hybridise selectively to rs2032583 or rs2235015 - see Uhr et al Neuron 57, pp203-209, 2008), the Corticotropin Releasing hormone Receptor 1 gene (CRHR1; eg the nucleic acid may hybridise selectively to rs878886) or the Vasopressin 1B Receptor gene (AVPR1B; eg the nucleic acid may hybridise selectively to rs28632197, see Keck et al. AJ Med Gen Part B, NeuroPsychi Res, 147B(7):1196-204, 2008).

The invention also includes a solid substrate for use in assessing the susceptibility of a subject to an anxiety disorder or depression, the solid substrate having attached thereto one or more nucleic acid molecules that hybridise selectively to a genomic region encompassing any one or more SNPs selected from the group consisting of rs3216799, rs6814934, rs7658048, rs2070950 and rs2070951 (eg with respective alleles '+CT', 'C', 'T', 'C' and 'C'), and that hybridise selectively to a genomic region encompassing one or more polymorphic sites in linkage disequilibrium with any one or more SNPs selected from the group consisting of rs3216799, rs6814934, rs7658048, rs2070950 and rs2070951 (eg with respective alleles '+CT', 'C', 'T', 'C' and 'C'). Preferences for the one or more polymorphic sites in linkage disequilibrium with any one or more SNPs selected from the group consisting of rs3216799. rs6814934, rs7658048, rs2070950 and rs2070951 include those described above.

It will be appreciated that the solid substrate of the Invention may have attached thereto at least 2, 3, 4, 5, 6, 7, 8, 9, 10, 11, 12, 13, 14, 15, 16, 17, 18, 19, 20, 21, 22, 23, 24, 25, 30, 35, 40, 45, or 50 different nucleic acid molecules.

It is also appreciated that the one or more nucleic acid molecules of the solid substrate may hybridise selectively to a region of genome at or close to the given polymorphic sites.

Typically, the solid substrate of the invention has attached thereto less than 100 different nucleic acid molecules, eg less than 95, 90,65. 80. 75, 70, 65, 60, 55, 50, 46, 40, 35, 30, 25, 20, 15 or 10 different nucleic acid molecules.

Typically, the one or more nucleic acid molecules of the solid substrate are less than 100 bases in length, such as less than 90, 80, 70, 60, 50, 40 or 30 bases. For example, the one or more nucleic acid molecules may be between 10 and 30 bases in length, such as 11, 12, 13, 14, 15, 16, 17, 18, 19, 20, 21, 22, 23, 24, 25, 28, 27, 28 or 29 bases in length.

It will be appreciated that the methods of the invention, and the uses, kits and solid substrates (eg DNA chips) described herein, may be used to determine the optimal therapy (eg pharmaco- or cognitive) for an anxiety disorder or depression. For example, clinical studies may be conducted in which treatment efficacy in patients is stratified according to genotype. In this way, the methods, uses, kits and solid substrates (eg DNA chips) of the invention may be useful in selecting subjects who may benefit from particular treatments for combating an anxiety disorder or depression.

It Will be appreciated that the methods, uses, kits and solid substrates (eg DNA chips) may also find uses in selecting cohorts of subjects for clinical trials.

A seventh aspect of the invention provides a method of combating an anxiety disorder or depression in a subject, the method comprising assessing the susceptibility of a subject to an anxiety disorder or depression according to the method of the first aspect of the invention and depending upon the outcome of the assessment treating the subject

By 'combating' we include the meaning that the invention can be used to alleviate symptoms of the disorder (ie palliative use) or to prevent the disorder or to treat the disorder.

In one embodiment, treating the subject comprises administering any one or more of an anti-depressant, an anti-convulsant, a beta-blocker, cortisol, a cortisol agonist, a cortisol antagonist, or an agent that modulates MR-expression to the subject. Examples of agents that modulate MR-expression include antidepressants such as tricyclic antidepressants (TCAs) and selective serotonin reuptake inhibitor (SSRIs), which have been shown to Increase MR expression (de Koet, DeRljk, Meijer, Clinical Practice article 08). Further examples include ACTH (adrenocorticotrophic hormone) which has been shown to increase MR expression in an animal model; steroids (both natural and synthetic); progesterone; and estrogen.

In animal models, acute and chronic stress is known to change MR expression, and it is possible that cognitive behavioural therapy and exercise affect MR expression. Thus, it is appreciated that treating the subject may comprise treating with a cognitive behavioural therapy or exercise regime.

The invention provides a compound for use in combating an anxiety disorder or depression in a subject who has been assessed as having, or having an increased likelihood of developing, an anxiety disorder or depression according to the first aspect of the invention, the compound being selected from an anti-depressant, an anti-convulsant, a beta-blocker, cortisol or an agent that modulates MR-expression.

The invention provides a use of a compound in the manufacture of a medicament for combating an anxiety disorder or depression in a subject who has been assessed as having, or having an increased likelihood of developing, an anxiety disorder or depression according to the first aspect of the invention, the compound being selected from an anti-depressant, an anti-convulsant, a beta-blocker, cortisol or an agent that modulates MR-expression.

Preferences for the subject are as defined above with respect to the first aspect of the invention. Preferably, the subject is a female human.

An eighth aspect of the invention provides an isolated polynucleotide comprising an MR gene sequence having a polymorphic site (eg SNP) at position 149585620 as numbered in Figure 4 (see position represented by SNP x in Figure 4 which is 8158 nucleotides before the translation start site (first ATG)). Preferably the polynucleotide has a 'T' allele at position 149585620 as numbered in Figure 4, which the inventors have shown resides on a haplotype comprising rs3216799, rs6814934, rs7858048, rs2070950 and rs2070951 with respective alleles '+CT', 'C', 'T', 'C' and 'C'.

In one embodiment, the polynucleotide comprises the sequence GAGGG[T]GTGAC, wherein the T in square brackets corresponds to the base at position 149585620 as numbered in Figure 4, or a sequence with at least 70% sequence identity to the sequence GAGGGF[T]GTGAC, for example at least 75% or 80% or 85% or 90% sequence identity to the sequence GAGGG[F]GTGAG, which has a 'T' at position 149585620 as numbered in Figure 4.

For example, the polynucleotide may comprise any of the sequences TGAGGG[T]GTGACC,
GTGAGGG[T]GTGACC, CGTGAGGG[T]GTGACCC or TCGTGAGGG[T]GTGACCCG, or the sequence in Figure 4 wherein the base at position 149585620 as numbered in Figure 4 is a 'T', or a sequence with at least 60%, 65%, 70%, 75%, 80%, 85%, 90%, 95%. 96%, 97%, 98% or 99% sequence identity to any of said sequences.

Preferably, the MR gene sequence is a human MR gene sequence.

A ninth aspect of the invention provides an isolated polynucleotide that selectively hybridises to the polymorphic site at position 149585620 as numbered in Figure 4. For example, the polynucleotide may hybridise to one allele of the polymorphic site (eg SNP) but not to the other allele of the polymorphic site (eg SNP) at position 149585620 as numbered in Figure 4. Thus, whether or not the polynucleotide hybridises to a genomic region encompassing the polymorphic site at position 149585620 as numbered in Figure 4 can be used as an indicator of which allele is present at that site.

Typically, the polynucleotides of the eighth and ninth aspects of the invention are less than 1000kb in length, for example no more than 900kb, 800kb, 700kb, 600kb, 500kb, 450kb, 400kb, 350kb, 300kb, 250kb, 200kb, 150kb, 100kb, 50kb, 40kb, 30kb, 20kb, 10kb, 9kb, 8kb, 7kb, 6kb, 5kb, 4kb, 3kb, 2kb or 1kb in length. In further embodiments, such polynucleotides are no more than 950b, 900b, 850b, 800b, 750b, 700b, 650b, 600b, 550b, 500b, 450b, 400b, 350b, 300b, 250b, 200b, 150b or 100b bases in length, such as less than 90, 80, 70, 60, 50, 40 or 30 bases. For example, the polynucleotides may be between 10 and 30 bases in length, such as 11, 12, 13, 14, 15, 16,17,18, 19. 20, 21, 22, 22, 23, 24, 25, 26, 27, 28 or 29 bases in length.

Typically, the polynucleotides of the eight and ninth aspects of the Invention are genomic DNA or cDNA

It is appreciated that the polynucleotides of the eighth and ninth aspects of the invention may be primers or probes, for example for use in the above methods, kits and solid substrates, to determine whether a subject has a particular allele (eg 'T') at the polymorphic site at position 149585620 as numbered in Figure 4. Accordingly, the Invention provides a polynucleotide according to the eighth or ninth aspect of the invention for use in assessing the susceptibility of a subject to anxiety disorder, wherein reduced susceptibility is indicated when the allele of the polymorphic site at position 149585620 as numbered in Figure 4 is T.

The invention also provides a polynucleotide according to the ninth aspect of the Invention for use in assessing the susceptible of a subject to an anxiety disorder or depression, wherein reduced susceptibility is indicated when the allele of the polymorphic site at position 149585820 as numbered in Figure 4 is 'T'.

The invention provides a use of a polynucleotide according to the ninth aspect of the invention in the manufacture of a reagent for assessing the susceptibility of a subject to an anxiety disorder or depression, wherein reduced susceptibility is indicated when the allele of the polymorphic site at position 149586620 as numbered in Figure 4 is 'T'.

The invention provides any novel method of assessing susceptibility to an anxiety disorder in a subject as herein disclosed.

The invention provides any novel kit of parts as herein disclosed.

The invention will now be described in more detail with the aid of the following Figures and Examples.

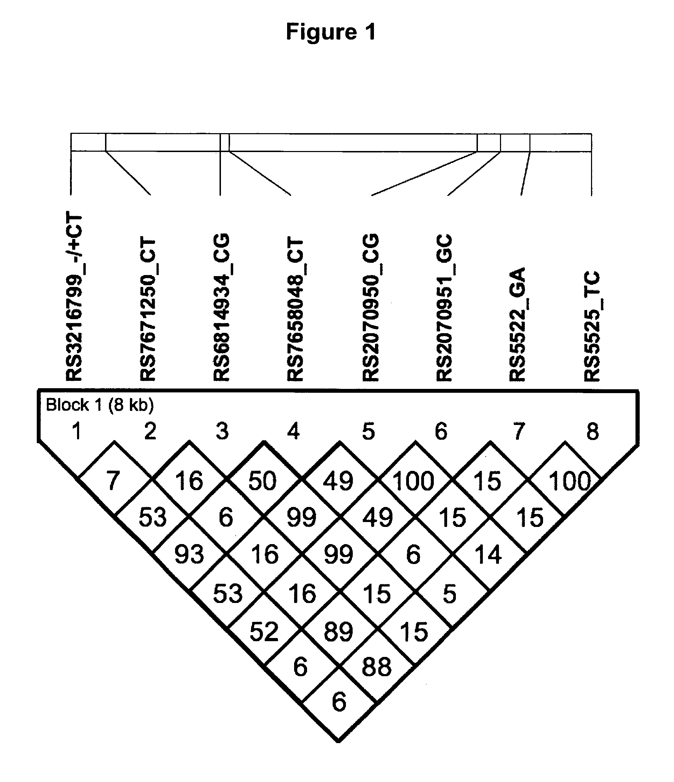

Figure 1. LD plot of the eight genotyped MR SNPs_{,} generated by Haploview. The SNPs are located in a region spanning 8 kb, starting in promoter region 2 and ending in exon 2. The magnitude of inter-marker LD scores is indicated in r2. All SNPs are enclosed in one haplotype bin. The SNP rs7871250 was highly linked to the functional MR I180V SNP (rs5522), and the SNP rs6814934 was highly linked to the functional MR -2G/C SNP (rs2070951).
Figure 2. Schematic overview of the MR- and GR gene structures with their respective haplotypes and haplotype frequencies. A. The gene encoding the MR consists of ten exons, exons la, exon 1β, till exon 9. The exons 1a and 1β result in two mRNA splice variants, MRa and MRβ. The exons 1α and 1β, the first 2 nucleotides of exon 2, and part of exon 9 (UTR) are not translated Into protein (light gray). The eight SNPs that were genotyped are indicated with arrows. The functional MR -2 G/C SNP (rs2070951) is located in exon 2, two nucleotides before the first translation start site. The functional MR I180V SNP (rs5522) is located in exon 2 and results in an Isoleucine to Valine amino-acid change (DeRijk, Wust et al (2008)). Both SNPs are located in a haplotype bin that extends into the promoter region. Three main haplotypes were found (plus five minor haplotypes with frequencies smaller than .02, not presented here, that were pooled with haplotype 2 as they had the same alleles for the -2 G/C and 1180V SIVPs). P1= promoter 1 in front of MR exon 1α, P2= promoter 2 in front of MR exon 1β. UTR= untranslated region. B. The gene encoding the GR consists of 17 exons, with the untranslated exons 1A-1H and 9α and 9β resulting in different mRNA splice variants. The five SNPs that were genotyped are indicated with arrows. Six haplotypes were found with frequencies similar as previously reported (van Rossum, Roks et al (2004); Derijk, van Leeuwen et a/ (2008)).

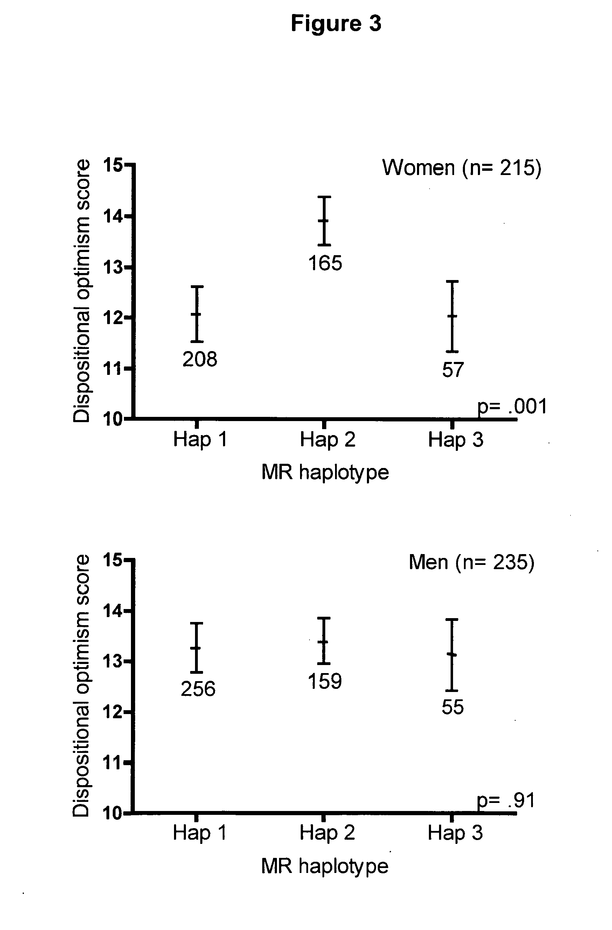

Figure 3. Comparison of crude mean (± SEM) dispositional optimism scores for the three most frequent MR haplotypes, separately for women and men. Only in women haplotype 2 was associated with an increased mean optimism score of almost 2 points as compared to haplotypes 1 and 3. In men mean optimism scores were similar between haplotypes 1 to 3. The number of chromosomes per haplotype group is indicated. Note that the scale for optimism on the y-axis is from 10 to 15. ANOVA was used to yield p-values for the overall comparison between the three haplotypes.
**Figure 4**. Nucleotide sequence of genomic region encompassing haplotype 3. Exons are marked in bold type and the positions of the eighteen SNPs within haplotype 2A are highlighted. The possible alleles of each SNP are also provided.
**Figure 5**.
   (A) SNPs linked to the SNPs reported in our studies, based on the hapmap database, subjects from Europe: The SNPs related to our SNPs are positioned in block 2, SNPs 60-82; chr4:149532194.,149632193. (B) Different alleles for 18 distinct SNPs in the MR gene that are linked to each other. There are three combinations of alleles that occur the most (haplotypes 1A-3A). The haplotype structure is based on SNPs used in the described association studies with optimism and LEIDS-R (rs3216799; rs7671250; rs6814934; rs7658048; rs2070950; rs2070951; rs5522; rs5525) + SNPs linked to those SNPs based on other Dutch cohorts (rs9992256; SNP x; rs2248038) + SNPs linked to those SNPs based on the hapmap database, subjects from Europe (rs2070949; rs11730626; rs11099695; rs11979719; rs2172002; rs4835519). Genotype data from the hapmap database were downloaded and analysed in the program Haploview (Barrett et al (2005) "Haploview:analysis and visualisation of LD and haplotype maps" Bioinformatics 21(2): 263-5) to reconstruct haplotypes based on genotype data from multiple subjects.

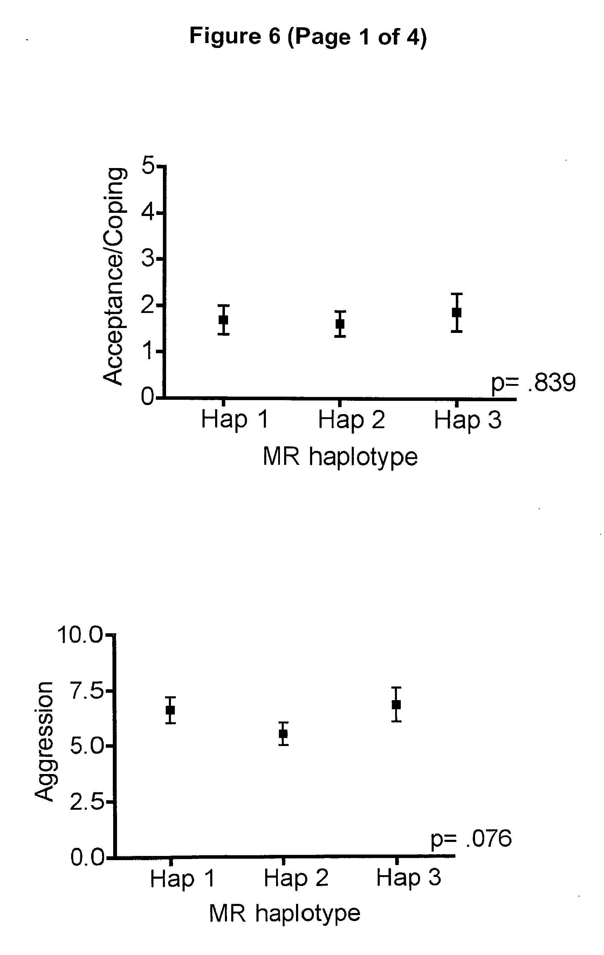

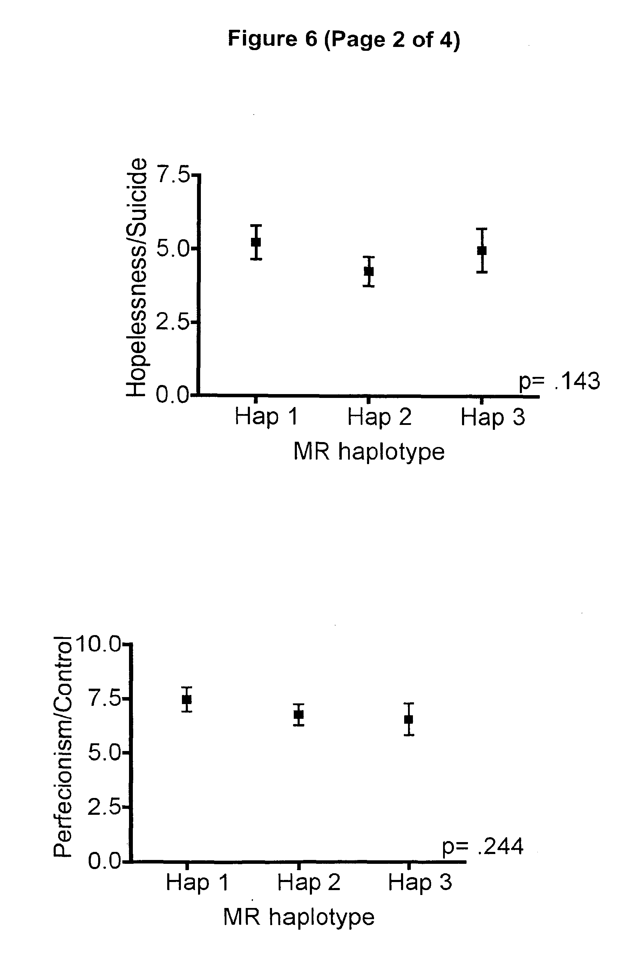

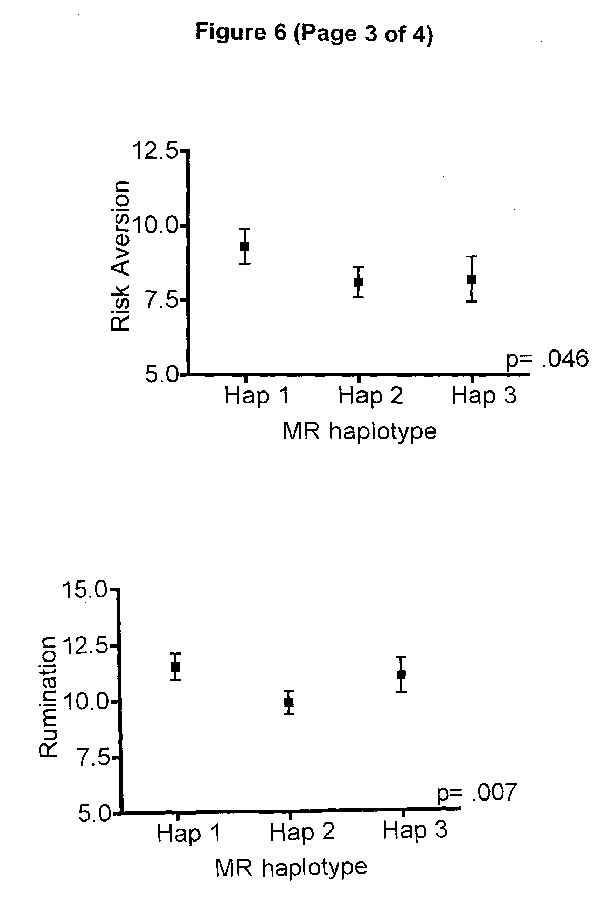

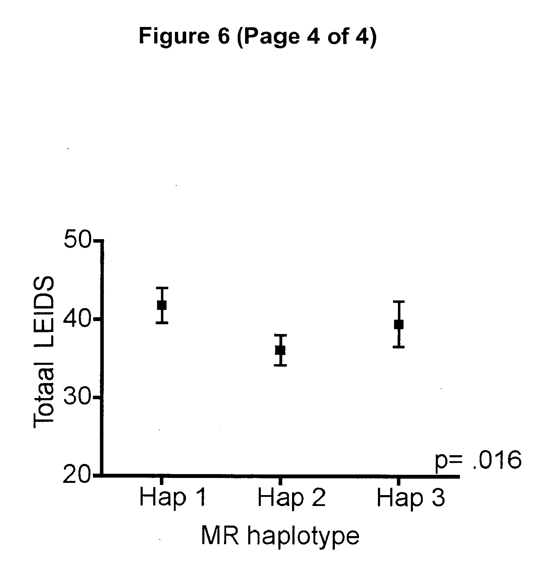

**Figure 6**. Scores for the total LEIDS-R and Its subscales for MR haplotypes 1-3 in the total group (n= 140). P-values represent results for ANOVA. P-value for linear regression analysis for haplotype 2, while correcting for sex, age and emotional abuse, was <.01 for the scale Rumination; p<.05 for Total LEIDS.

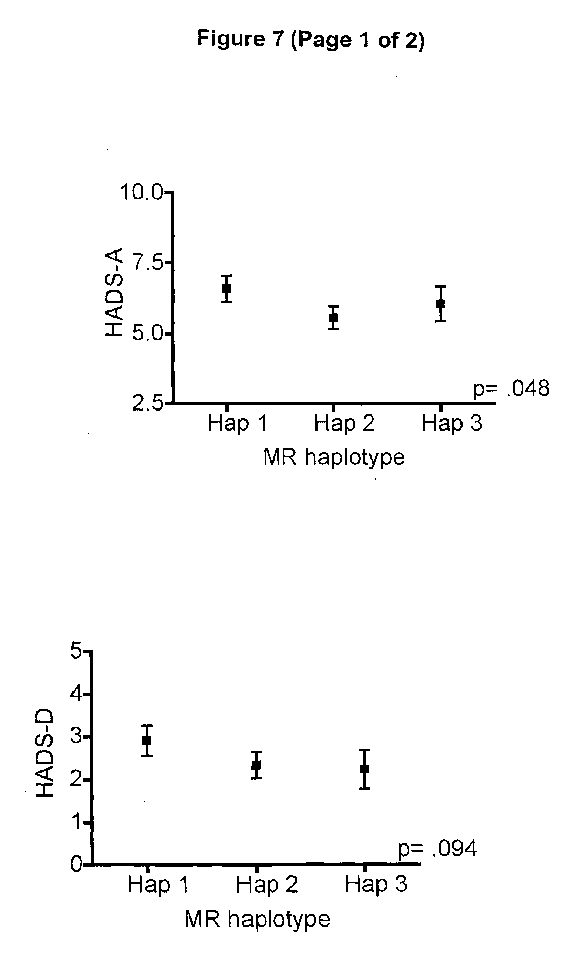

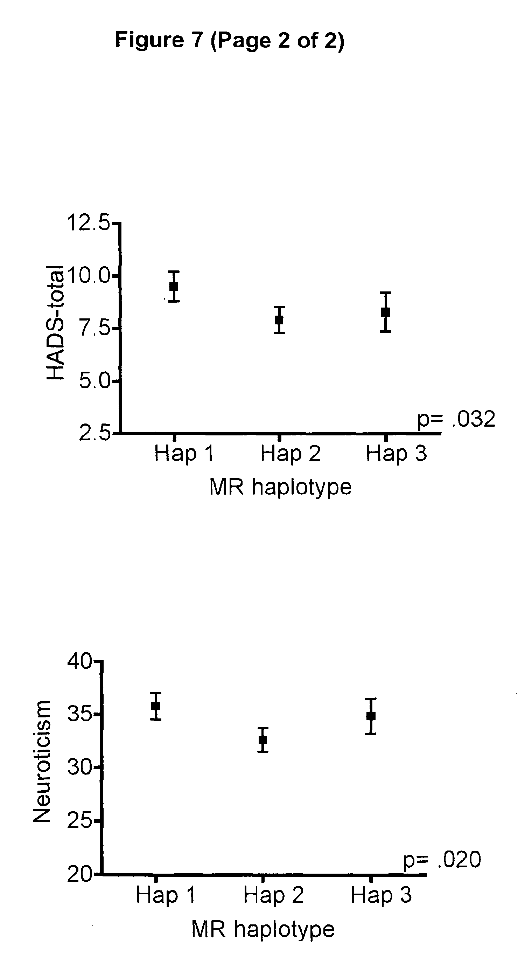

**Figure 7**. Scores for neuroticism, symptoms for anxiety and depression in the total group. Scores for the HADS-A, HADS-D, total HADS and neuroticism (NEO-PI) for MR haplotypes 1-3 In the total group (n= 140). P-values represent results for ANOVA. None of the scales gave a significant association with the haplotypes with linear regression analysis.

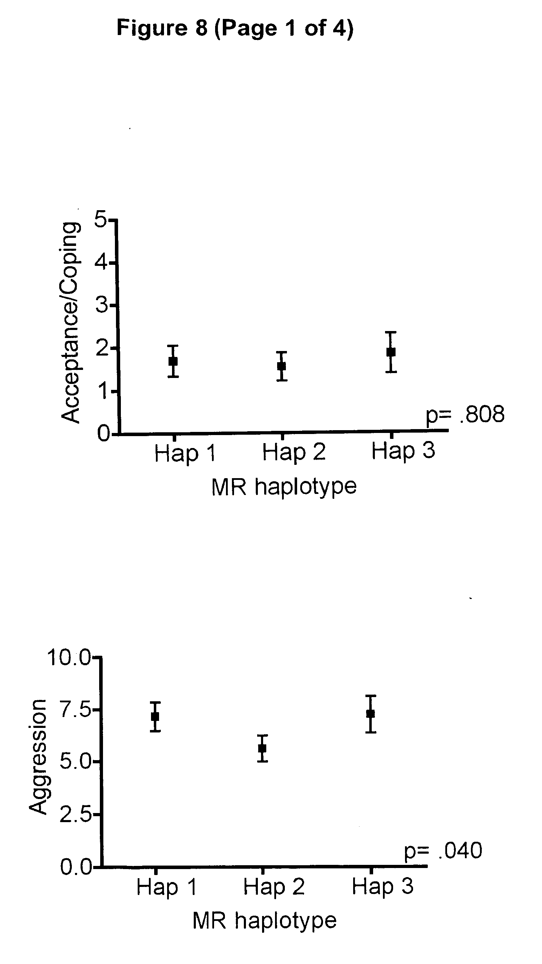

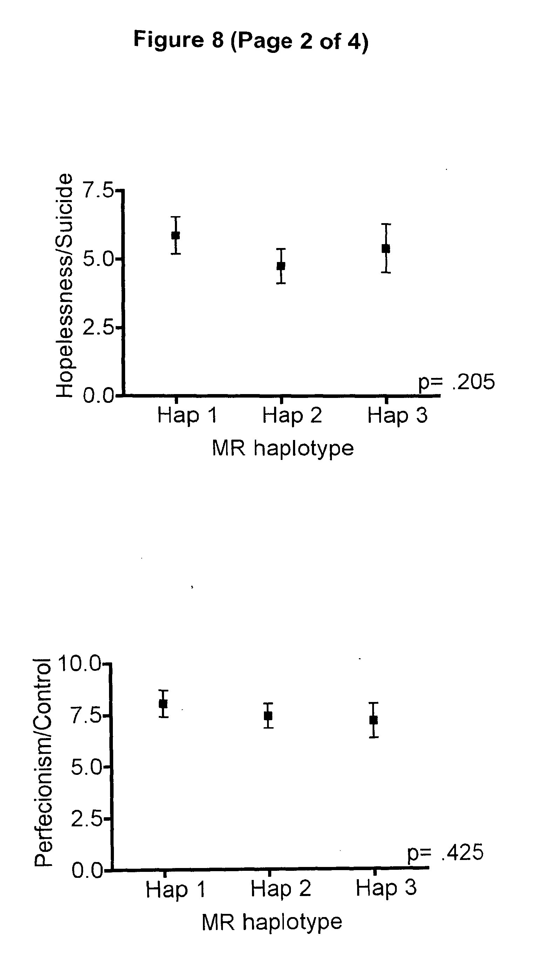

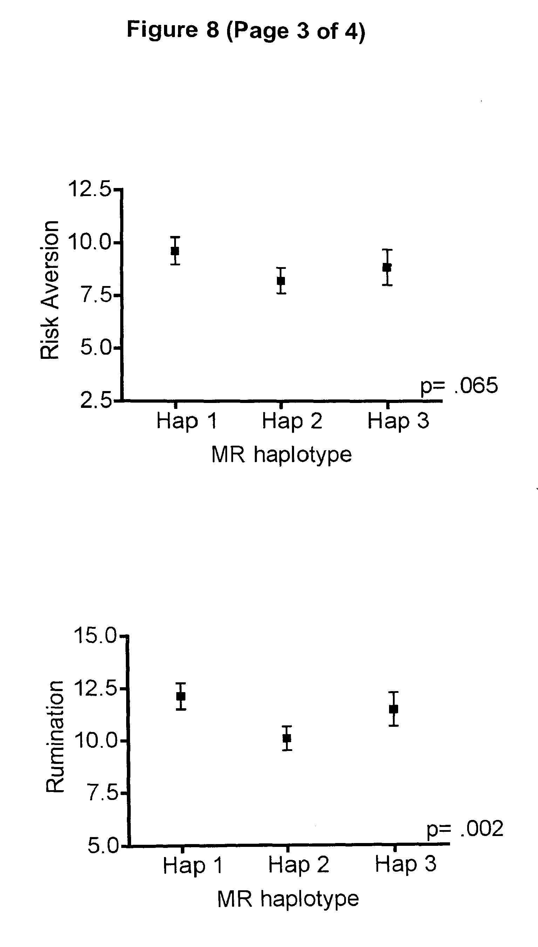

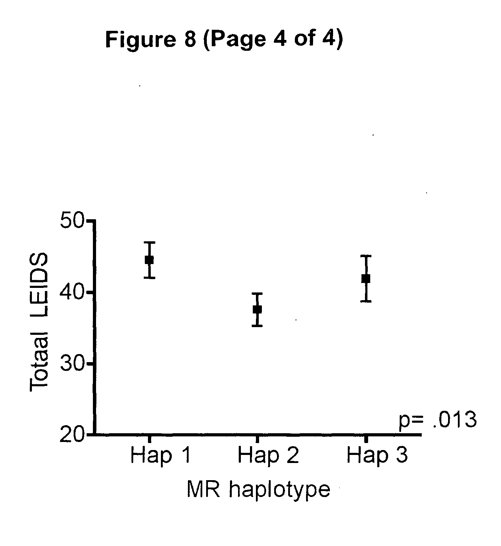

**Figure 8**. Scores for the total LEIDS-R and its subscales for MR haplotypes 1-3 in females only (n= 97). P-values represent results for ANOVA. P-value for linear regression analysis for haplotype 2, while correcting for age and emotional abuse, was <.05 for the scales Aggression, Hopelessness,Risk aversion; p< .001 for Rumination; p<.01 for Total LEIDS.

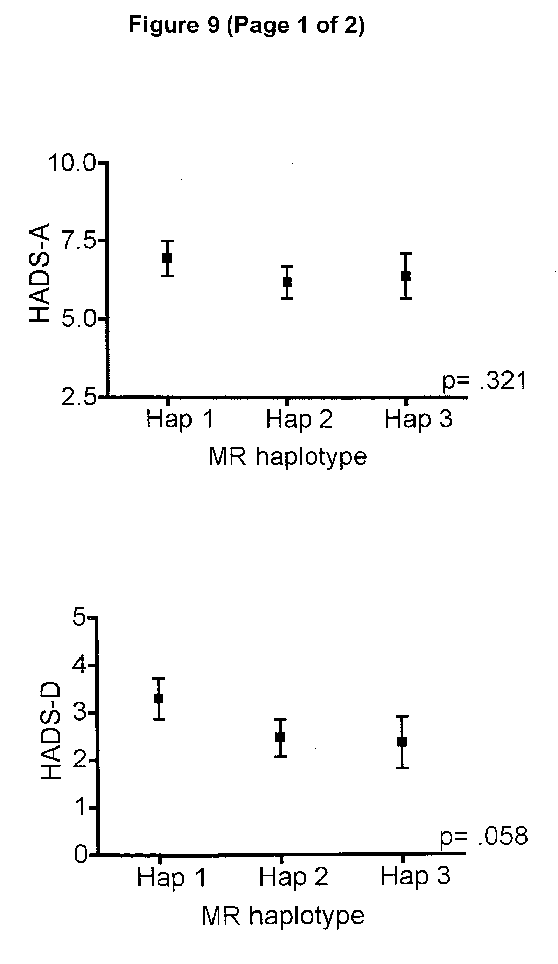

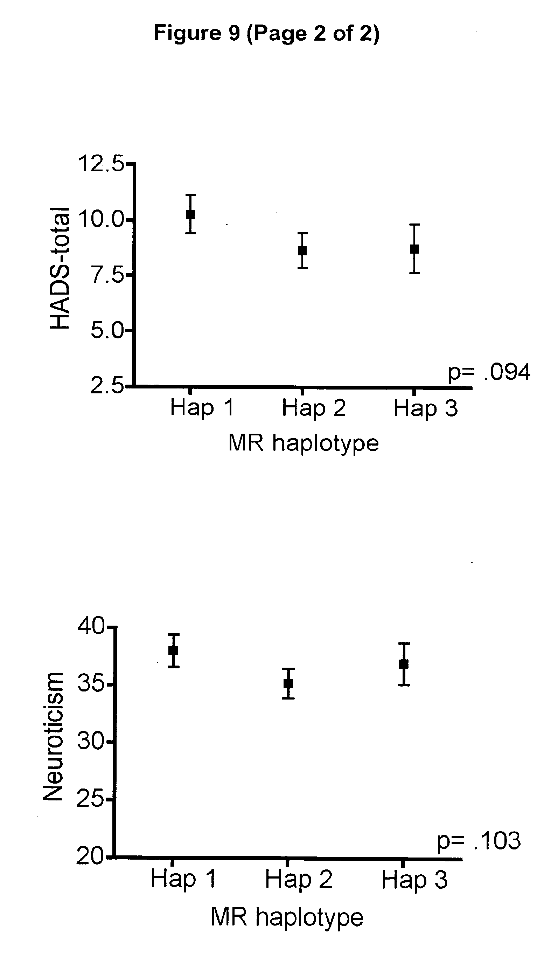

**Figure 9**. Scores for the HADS-A, HADS-D. total HADS and neuroticism (NEO-PI) for MR haplotypes 1-3 in females only (n= 97). P-values represent results for ANOVA. P-value for linear regression analysis for haplotype 2, while correcting for age and emotional abuse, was < .05 for the scales HADS-D, HADS-total, Neuroticism.

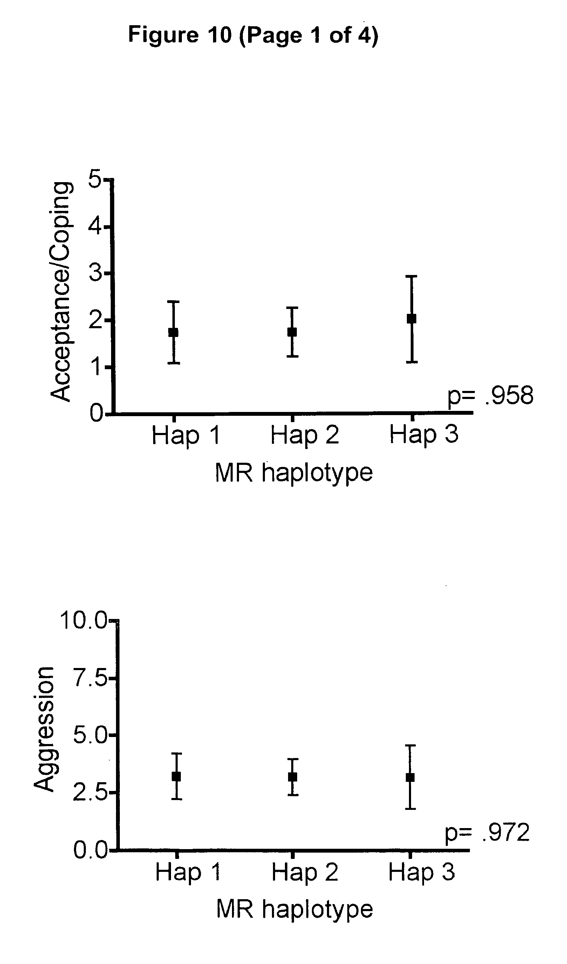

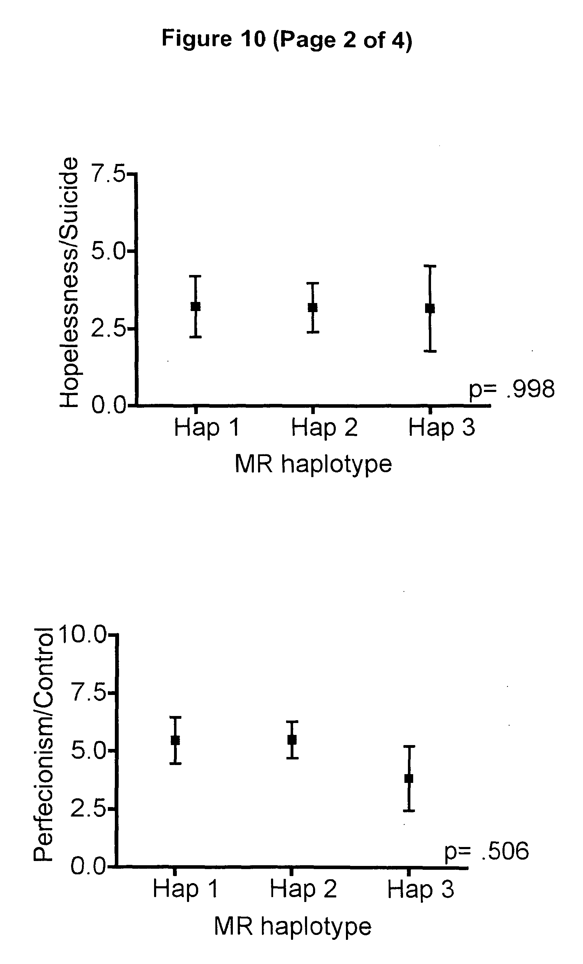

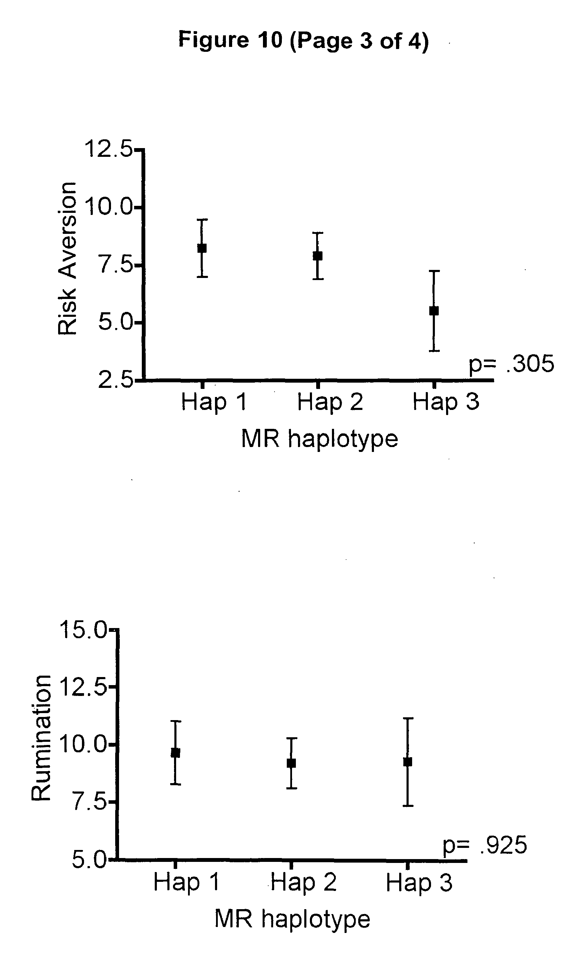

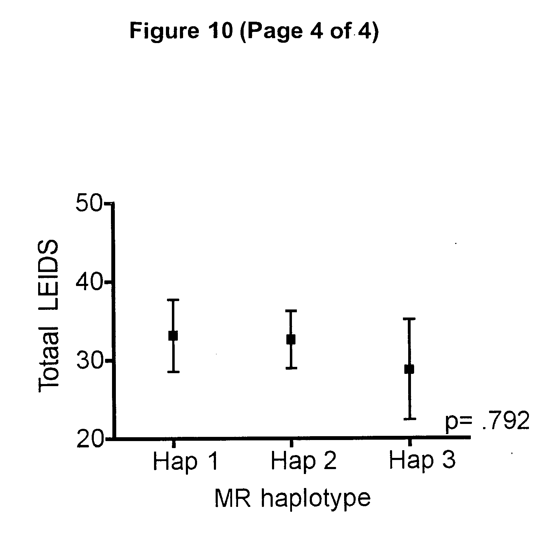

**Figure 10**. Scores for the total LEIDS-R and its subscales for MR haplotypes 1-3 in males only (n= 43). P-values represent results for ANOVA. None of the scales gave a significant association with the haplotypes with linear regression analysis.

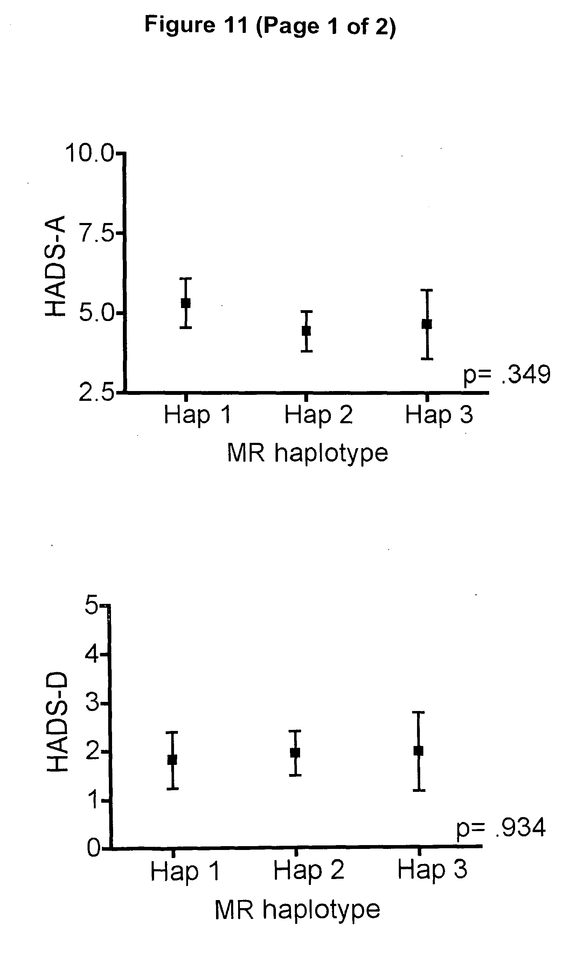

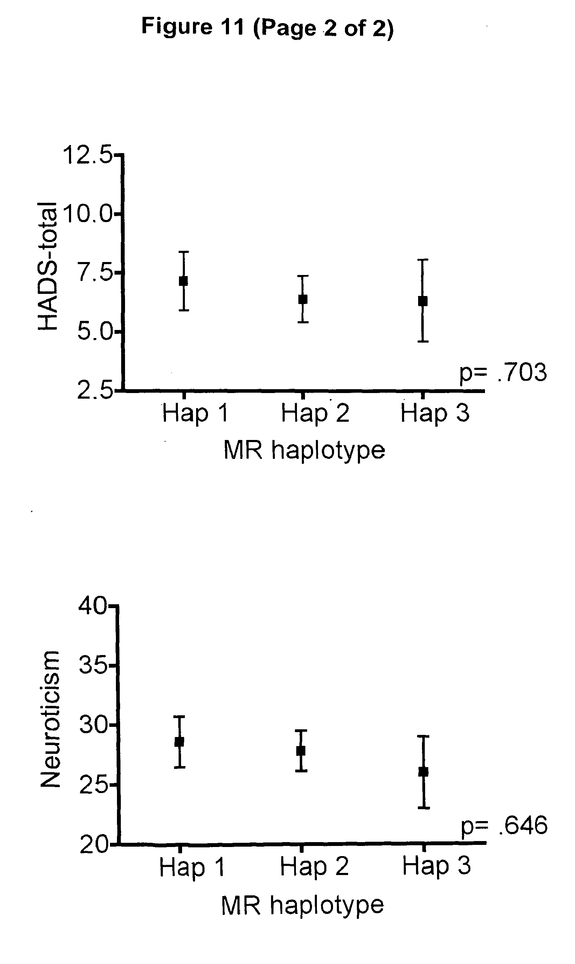

**Figure 11**. Scores for neuroticism, anxiety and depression in male students. Scores for the HADS-A, HADS-D, total HADS and neuroticism (NEO-PI) for MR haplotypes 1-3 in males only (n= 43). P-values represent results for ANOVA. None of the scales gave a significant association with the haplotypes with linear regression analysis.

### EXAMPLE 1: A mineralocorticoïd receptor haplotype is associated with dispositional optimism in elderly women but not in men

### Summary

The brain mineralocorticoid receptor (MR), together with the glucocorticoid receptor (GR), mediates the effects of the hormone cortisol on behaviour and cognition. We have tested the relation between MR gene variants and dispositional optimism. Dispositional optimism is defined as having generalized expectancy of positive outcomes for the future. It is a rather stable personality trait and might confer resilience against depression.

Eight single nucleotide polymorphisms (SNPs) in the MR gene, including two functional MR SNPs, were genotyped In 450 subjects aged 65-85 of the Dutch Arnhem Elderly Study. Six known GR haplotypes, constituted of five SNPs, were taken along. Participants completed a questionnaire on their subjective levels of dispositional optimism as part of the 'Scale of Subjective Well-being for Older persons' (SSWO). Haplotype reconstruction resulted in 3 main MR haplotypes with frequencies of .52, .36, and .12. MR haplotype 2 was associated with higher levels of optimism (15% increase) in women (*p*< .001) but not in men (*p*= .85; *p*= .01 for interaction). The effect persisted after correction for several potential confounders and was estimated to explain 6% of the variance in optimism. The GR gene haplotypes had no influence on optimism scores. To conclude, our results suggest that MR haplotype 2 is associated with higher levels of dispositional optimism in women, which may establish resilience against stress and depression.

### Introduction

The mineralocorticoid receptor (MR) is initially known for its function in the kidney, mediating aldosterone effects on salt status. Yet importantly, the MR is also expressed in the brain, mainly in limbic structures and frontal cortex. It has a ten-fold higher affinity for the hormone cortisol - the main corticosteroid - than its regulatory partner, the glucocorticoid receptor (GR). Central MR Is involved In basal activity of the hypothalamic-pituitary-adrenal (HPA) axis, autonomic outflow and in physiological response to a stressor (De Kloet, Vreugdenhil *et al* (1998); de Kloet, Van Acker *et al* (2000)). In humans, associations have been found between MR- and GR gene variants and HPA activity (Derijk, van Leeuwen *et al* (2008)). A single nucleotide polymorphism (SNP) in the MR gene, the MR -2 G/C SNP, was associated with basal cortisol levels in elderly (Kuningas, de Rijk *et al* (2007)). In a study among healthy young males, another variant, the MR I180V SNP, modulated cortisol response after a psychosocial stressor (Trier Social Stress Test, TSST) (DeRljk, Wust *et al* (2006)). This same variant was also related to more feelings of depression in elderly (Kuningas, de Rijk *et al* (2007)). Moreover, associations between MR- and GR gene variants and stress-reactivity were found to be gender specific (Kumsta, Entringer *et al* (2007); Wust, Kumsta *et al* (2009))(Nienke PNE09 in press). These gender specific effects of MR and GR gene variants might contribute to the differences in prevalence of depression between men and women.

In addition to regulation of the HPA axis, central corticosteroid receptors are responsible for the effects of cortisol on behaviour, learning and memory (Oitzl and de Kloet (1992); De Kloet, Vreugdenhil *et al* (1998)). Interestingly, based on studies with rodents the MR has been identified as a mediator of emotions and of explorative and coping behaviour (Oitzl) and de Kloet (1992); Conrad, Luplen *et al* (1997); Rozeboom, Akil *et al* (2007)). Following an environmental demand the MR, but not the GR, regulates acute response selection, aimed to cope In an adaptive way. Also in humans cortisol and its receptors are necessary for learning and memory (Lupien, Wilkinson *et al* (2002)). However, it is unknown whether the MR and GR modulate human coping behaviour or psychological characteristics. This would be interesting to know, as psychological traits influence coping behaviour and eventually can establish resilience or vulnerability to psychopathology (Carver and Connor-Smith 2009).

Dispositional optimism is a positive personality characteristic that seems relatively stable over time and its heritability is estimated at 25-40% (Plomin, Scheier *et al* (1992); Scheier, Carver *et al* (1994); Giltay, Kamphuls *et al* (2006)). The construct of dispositional optimism was introduced in 1985 by Scheier and Carver and was described as having generally positive outcome expectancies (Scheier and Carver 1985). It is associated with enhanced goal engagement and self-regulatory flexibility when encountering environmental demands or stressful situations (Scheler, Weintraub *et al* (1986); Carver, Pozo *et al* (1993); Nes and Segerstrom (2006); Geers, Wellman *et al* (2009)). Eventually, this can be beneficial for physiological and psychological health. Dispositional optimism is associated with less distress and predicts lower risk for depression and all-cause and cardiovascular death (Plomin, Scheier *et al* (1992); Scheler and Carver (1992); Carver, Pozo *et al* (1993); Scheier, Carver *et al* (1994); Vickers and Vogeltanz (2000); Giltay, Geleijnse *et al* (2004); Giltay, Kamphuis *et al* (2006); Giltay, Zitman *et al* (2006)). Interestingly, variability in levels of optimism and positive affect seems to relate to differences in basal cortisol levels (Lai, Evans *et al* (2005); steptoe, O'Donnell *et al* (2008)).

We hypothesised that the MR influences dispositional optimism, possibly modified by sex. In order to test this, we have analysed the association of eight MR gene variants, including two known functional MR SNPs, with optimism that was measured in subjects of the Arnhem elderly study cohort. Dispositional optimism was assessed with the 'scale of Subjective Well-being for Older persons' (SSWO), a questionnaire measuring subjective wellbeing in elderly (Tempelman (1987)). In addition, genotypes for five common GR variants were determined.

### Methods

### Study population

Our study population was based on the Arnhem Elderly Study, a population-based cohort study that started in 1991-1992. The study design and population characteristics have been described previously (van den Hombergh, Schouten *et al* (1995)). The subjects that we included in our research were part of a random sample that was followed for 9.1 years to assess the relation between a person's level of dispositional optimism and all-cause and cardiovascular mortality (Giltay, Geleijnse *et al* (2004)). This sample included men and women with an age between 65 to 85 years old who were independently living in the city of Arnhem, the Netherlands. Of this random sample of 1793 individuals, 1012 subjects gave an interview, 685 subjects underwent a physical examination, and 641 subjects gave a blood sample. Of the 641 blood samples, 499 (77.8%) DNA isolates were available for genotyping, which was successful for 473 (94.8%) DNA samples. The final subset of 450 subjects (optimism scores were missing for 23 subjects) did not differ from the Initial group of 1012 subjects that gave an interview on sex, education, body mass index (BMI), or total number of chronic diseases. The included subjects were, however, significantly younger (mean age 73.7 ± 5.7 vs. 75.2 ± 5.7, *p*< .001), more often together (60.9% vs. 54.1%, *p*= .03), more often had a higher socioeconomic status (SES; 64.0% vs. 55.8%, *p*< .01), more often suffered from cardio-vascular disease (CVD; 24.0% vs. 14.9%, *p*=.01),and were more optimistic (mean score 13-40 ± 4.68 vs. 12.36 ± 4.91, *p*= .001). When comparing the 450 subjects with the 49 subjects for whom we did not have a complete dataset, no significant differences were found for any of the sociodemographic or health factors. This study was approved by the Medical Ethics Committee of Wageningen University (Wageningen, the Netherlands). All participants provided written informed consent

### Assessment of dispositional optimism

Optimism was assessed using the Dutch Scale of Subjective Well-being for Older Persons (SSWO) developed by Groningen University (Groningen, the Netherlands) (Tempelman (1987)). The SSWO consists of five subscales including health, self-respect morale, contacts, and optimism. Validity of the SSWO was previously assessed by comparing the results with objective measures of well-being (eg physical activity, mobility, use of health care, and activities of daily living) and the Hopkins Symptom Checklist (Tempelman (1987)). For each subscale an individual could indicate to what extent it conforms to a particular statement on a 3-point scale (from 0 to 2). The seven questions of the optimism subscale were: "I often feel that life is full of promises", "I still have positive expectations concerning my future", there are many moments of happiness in my life", "I do not make any more future plans", "Happy laughter often occurs", "I still have many goals to strive for", and "Most of the time I am in good spirits" (our translations). The subscale had an adequate internal consistency (Cronbach's α: 0.76) and reliability (test-retest reliability coefficient: 0.76) (Tempelman (1987)). Questionnaires with missing data for the optimism subscale were excluded from the analyses. A mean item score for the optimism subscale was calculated and multiplied by 10, resulting in scores ranging from 0 to 20, with higher scores indicating a higher level of optimism.

### Demogrophics, health, and blood sampling

All data on demographics and health were assessed by trained interviewers (van den Hambergh, Schouten *et al* (1995); Giltay, Geleijnse *et al* (2004)). Dichotomous variables were created for sex (0= women; 1= men), marital status (0= living together as a married or unmarried couple; 1= otherwise), education (0= otherwise; 1= higher vocational or university), presence of CVD (0= absent; 1= present), and SES (0= housewives, unskilled and skilled workers, and lower employees; 1= small-business owners, employees, and higher professions; for married or widowed women SES was defined according to that of the husband). A variable for total number of chronic diseases coded for the total number of chronic disorders and illnesses of the respondent (0, 1, 2, 3, 4, or 5 or more from a list of 24; eg chronic gastric disease, cancer, thyroid disease). Body mass index (BMI) was calculated by dividing weight in kilograms (to the nearest 0.5 kg with the subject dressed but not wearing shoes) by height in meters squared (to the nearest 0.5 cm). A single blood sample was obtained from 641 subjects. Samples were stored at-80°C until further analysis.

### Genotyping

Genomic DNA was Isolated from the blood samples according to standard procedures. Genotypes were determined for the functional MR -2G/C (rs2070951) and I180V (rs5522) SNPs. Two SNPs, the rs2070950 and rs5525, were included as additional control SNPs in case of genotyping failure for the -2 SNP or I180 SNP respectively. Four additional SNPs, with the accession numbers rs3216799 (an insertion-deletion polymorphism of two nucleotides, CT), rs7671250, rs6814934 and rs7678048, which are located in the MR promoter region, were assessed. In addition, genotypes for several common GR variants, the T*th*/*III* (rs10052957), ER22EK (rs6189), N363S (rs6195), *Bcl*1 (rs41423247 and 9β (rs6198) SNPs, were assessed.

Genotyping was conducted using a Sequenom MassARRAY iPLEX assay (Sequenom, San Diego, CA, USA). After a 'touchdown' polymerase chain reaction (PCR) and a primer extension reaction to introduce mass-differences between alleles, reaction products were desalted, processed and mass differences were detected using an Autoflex (Bruker, Wormer, Netherlands) MALDI-TOF Mass Spectrometer. Genotypes were assigned real-time using MassARRAY TYPER Analyzer 3.4 software (Sequenom, San Diego, CA, USA). As quality control, 5 to 10% of the samples were genotyped in duplicate, and positive and negative controls that were included were consistent. Samples that failed for 50% of the SNPs or more were omitted from further analysis.

### Statistical analysis

Allele frequencies for the different SNPs were tested for Hardy-Weinberg Equilibrium (HWE) using HaploView (version 4.1 for Mac OSX) (Barrett, Fry *et al* (2005)). In addition, this program was used to test whether the SNPs for the MR gene were in linkage disequilibrium (LD) and to reconstruct haplotypes for the MR and GR genes. We used r² and D¹ to verify respectively the magnitude of inter-marker correlations and to define haplotype bins with the Solid Spine of LD method implemented in HaploView. Individual haplotypes were reconstructed in SNPHAP (version 1.3; available online at http://www-gene.cimr.cam.ac.uk/clayton/software/; last visited on Feb 14, 2008). For the MR, haplotypes were reconstructed based on only the -2 G/C and I180V SNPs, which tag haplotypes 1-3 (five minor haplotypes with frequencies below .02 were pooled with haplotype 2 with a frequency of .32, resulting In a total frequency of 36). Samples with probabilities below 0.50 (n=1) were discarded. For the GR gene, haplotypes with a probability below 0.50 were also discarded (n=8); haplotypes with probabilities between 0.50 and 0.95 (n=10) or above 0.95 were weighted for their probabilities in the statistical analyses. Further analysis was performed in SPSS, version 16.0 for Mac OSX (SPSS Inc., Chicago, IL, USA).

Association between dispositional optimism and sociodemographic or health factors was tested with regression analysis or an independent-samples t-test. Differences between men and women on these variables were tested using an independent-samples t-test or a χ² test. The main aim was to test the influence of MR haplotypes on the level of optimism. To verify whether any of the MR or GR SNPs was associated with optimism scores, one-way ANOVA was used. Subsequently, differences between the MR haplotypes were tested using linear regression analysis. Next, analyses were repeated for the GR haplotypes. Furthermore, confounding effects of the GR haplotypes on the results with the MR haplotypes was verified. Comparison of mean optimism scores for the different MR diplotypes was conducted with one-way ANOVA, followed by a post-hoc Gabriel test. In a second regression analysis, we adjusted for potential confounding effects of sex (when appropriate), age, educational level, marital status, and SES in multivariable model 1, or additionally for CVD and total number of chronic diseases in model 2. All regression analyses were repeated while stratifying the data for sex. Finally, as the optimism scores showed a somewhat negatively skewed distribution, scores were inversed and log-transformed (to approach a normal distribution); and tests were repeated with these log-transformed data. A two-sided *p*-value < .05 was considered statistically significant. As our main interest was the one test determining the association between the MR haplotypes and optimism, no Bonferroni correction was applied.

### Results

### Sample characteristics

Data sets with optimism scores, genotypes, sociodemographic and health-related variables were available for 450 individuals (Table 1; note that for SES, BMI, total number of chronic diseases, and CVD several data points were missing). Increasing age, lower educational level, living alone, and more chronic disease were significantly associated with lower dispositional optimism scores (*p's*< .05). No associations with optimism were found for SES, BMI, or CVD. There were important sex differences in sociodemographic and health-related variables (Table 1), but the mean optimism scores did not differ between men and women (*p*= .78). One subject reported a depressive disorder.

### MR and GR haplotype structure and frequencies

All allele frequencies of the MR and GR SNPs were in HWE (*ρ*> .10). For an overview of individual SNP genotype frequencies see Table 2. Allele frequencies of the MR -2G/C and 1180V SNPs were similar as previously reported (DeRijk, Wust *et al* (2006); Kuningas, de Rijk et al (2007)) Nienke PNE09 in press). Reconstruction of MR haplotypes resulted in one haplotype bin containing all eight genotyped SNPs (Figure 1). The inter-marker correlation between the functional MR -2G/C (rs2070951) and I180V (rs5522) SNPs was low (r²= 15), but these SNPs were in perfect LD with respectively the rs2070950 or rs5525 (r²= 1.0). Correlations between the SNPs in the promoter region and the -2G/C and I180V SNPs ranged from .05 to .99 and D' LD values among all eight SNPs ranged from .86 to 1-0. Figure 2A shows the structure of the three main MR haplotypes. For the GR gene inter-marker correlations (r²) were between .0 and .45, D' LD values were between .08 and 1.0. Similar frequencies were found for the six haplotypes that previously have been described (Figure 2B).

### Associations between individual MR or GR SNPs and dispositional optimism

For the eight MR SNPs, significant associations were found between dispositional optimism and the SNPs rs3216799, rs6814934, rs7658048, rs2070950 and rs2070951, with the highest significant associations found for the promoter SNPs rs3216799 and rs7658048 (Table 2). As sex differences exist for the effects of MR and GR SNPs on HPA activity, association analysis was repeated while stratifying for gender. Significant associations between optimism scores and the MR SNPs were found only in women, but not in men. No associations were found between dispositional optimism and any of the GR SNPs, not for the total group or for women or men separately.

### Associations between MR haplotypes and dispositional optimism

We also tested associations between the naturally occurring MR haplotypes and the level of dispositional optimism. The MR haplotypes were significantly associated with dispositional optimism scores; haplotype 2 was associated with higher optimism when compared to the baseline haplotype 1 (Table 3). This haplotype 2 contains the functional -2 C-allele, while it does not contain the 180 V-allele. Importantly, we found a strong MR haplotype 2-by-sex Interaction effect (*p*= .01). Only in women, haplotype 2 was related to higher levels of optimism, while no significant effect was found in men (Table 3 and Figure 3). Results were similar after adjustment for covariates in models 1 and 2, haplotype 2 giving an average increase per haplotype allele of 1.7 on a maximum score of 20 and explaining 6% of the variance in optimism (ΔR²= 0.06, Table 3). Comparing the mean optimism scores for the six different MR diplotypes showed a dear and significant (one-way ANOVA, *p*= .02) allele dose effect of haplotype 2 in women (data not shown). Post-hoc analysis revealed that the 2/2 diplotype was associated with significantly higher levels of dispositional optimism compared to the 1/1 diplotype, *p*< .01; the 1/2 diplotype, *p*= 01; and the 1/3 diplotype, *p*= .01.

No association was found between dispositional optimism scores and the GR haplotypes (*p*= .65 for the model for the total group), not in women (*p*= .57), not In men (*p*= .86;). In addition, including the GR haplotypes as confounders in the regression analysis for the MR haplotypes on optimism did not change the results. Finally, similar results were found when tests were repeated with the logarithmically transformed optimism data (data not shown).

The SSWO questionnaire actually consists of five subscales, namely health, self-respect, morale, contacts, and optimism. Association between the three MR haplotypes and these additional subscales was verified. Interestingly, also only in women haplotype 2 was associated with higher levels of self-respect, p< .01: and the total SSWO score, p< 0.001; a statistical trend was found for higher morale, p= .06 and better health p= 0,07.

**Table 1. Sociodemographic and health factor measures according to sex in 450 elderly subjects**

| **Variable** | | **Total** n | **Total** | **Women** | **Men** | ***p-value**** |
|---|---|---|---|---|---|---|
| **Gender** | | 450 | 450 | 215 (47.8%) | 235 (52.2%) | |
| **Age** | | 450 | 73.7±5.7 | 74.2±5.9 | 73.2±5.5 | 0.06 |
| **Education level** | Highschool or University | 450 | 91 (20.2%) | 26 (12.1%) | 65 (27.7%) | <.001 |
| | Otherwise | | 359 (79.8%) | 189 (87.9%) | 170 (72.3%) | |
| **Marital status** | Living together ((un)married couple) | 450 | 274 (60.9%) | 80 (37.2%) | 194 (82.6%) | <.001 |
| | Otherwise | | 176 (39.1%) | 135 (62.8%) | 41 (17.4%) | |
| **Socioeconomic status** | Low | 444 | 160 (36.0%) | 88 (41.7%) | 72 (30.9%) | 0.02 |
| | High | | 284 (64.0%) | 123 (58.3%) | 161 (69.1 %) | |
| **BMI** | | 448 | 25.8±3.9 | 26.3±4.5 | 25.4±3.1 | 0.01 |
| **Total number of chronic diseases** | 0 | 446 | 90 (20.2%) | 32 (15.0%) | 58 (25.0%) | 0.02 |
| | 1 | | 122 (27.4%) | 54 (25.2%) | 68 (29.3%) | |
| | 2 | | 100 (22.4%) | 51 (23.8%) | 49 (21.1%) | |
| | 3 | | 67 (15.0%) | 39 (18.2%) | 28 (12.1%) | |
| | 4 | | 30 (6.7%) | 14 (6.6%) | 16 (6.9%) | |
| | 5 or more | | 37 (8.3%) | 24 (11.2%) | 13 (5.6%) | |
| **Cardiovascular disease** | Absent | 446 | 339 (76.0%) | 171 (79.9%) | 168 (72.4%) | 0.06 |
| | Present | | 107 (24.0%) | 43 (20.1%) | 64 (27.6%) | |
| **Dispositional optimism** | | 450 | 3.40±4.68 | 13.46±4.69 | 13.34±4.68 | 0.78 |

| | | | | | | |
|---|---|---|---|---|---|---|
| Data are mean ± SD or n (%). *An independent-samples t-test or χ² test was used to examine p-values for sex differences. | | | | | | |

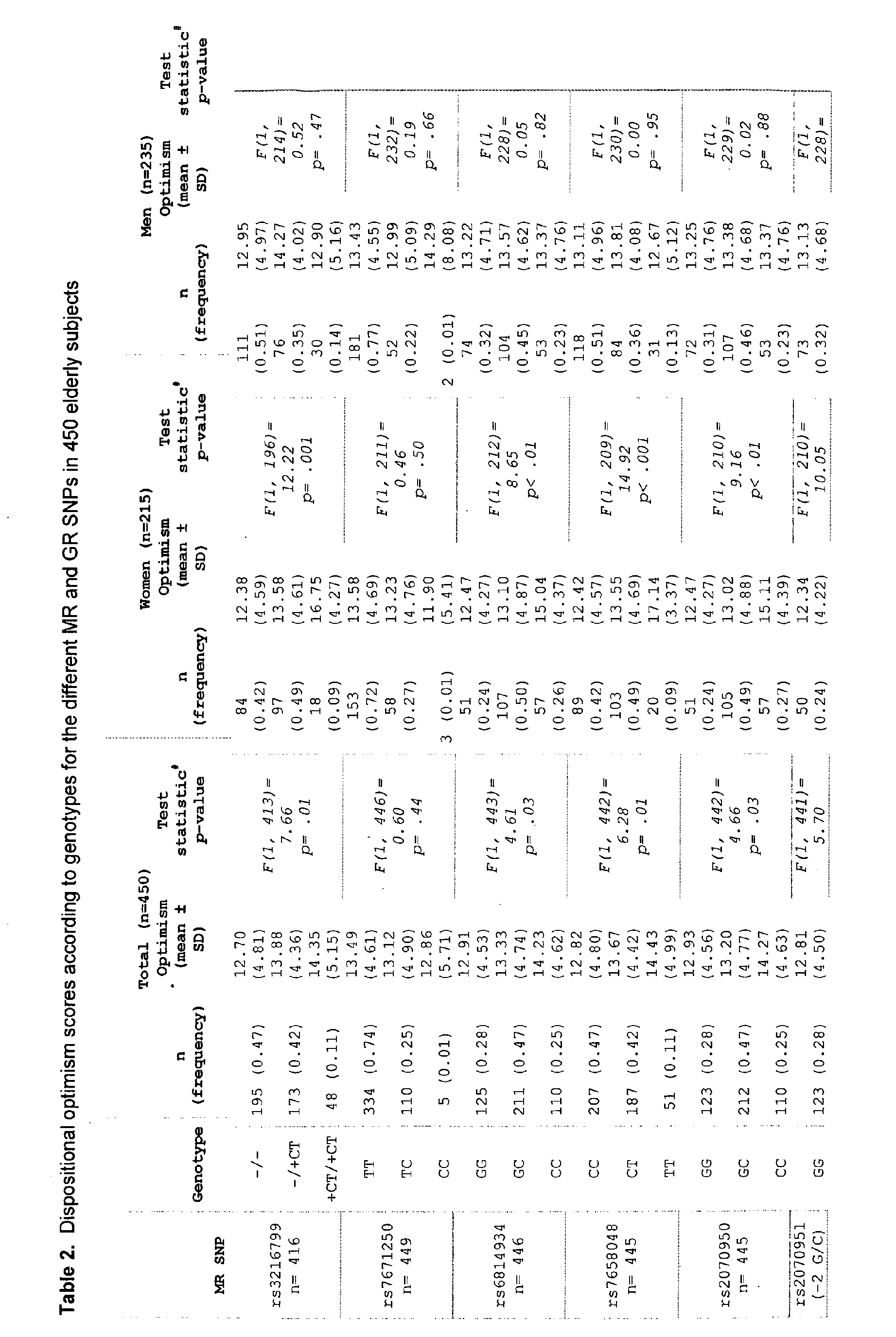

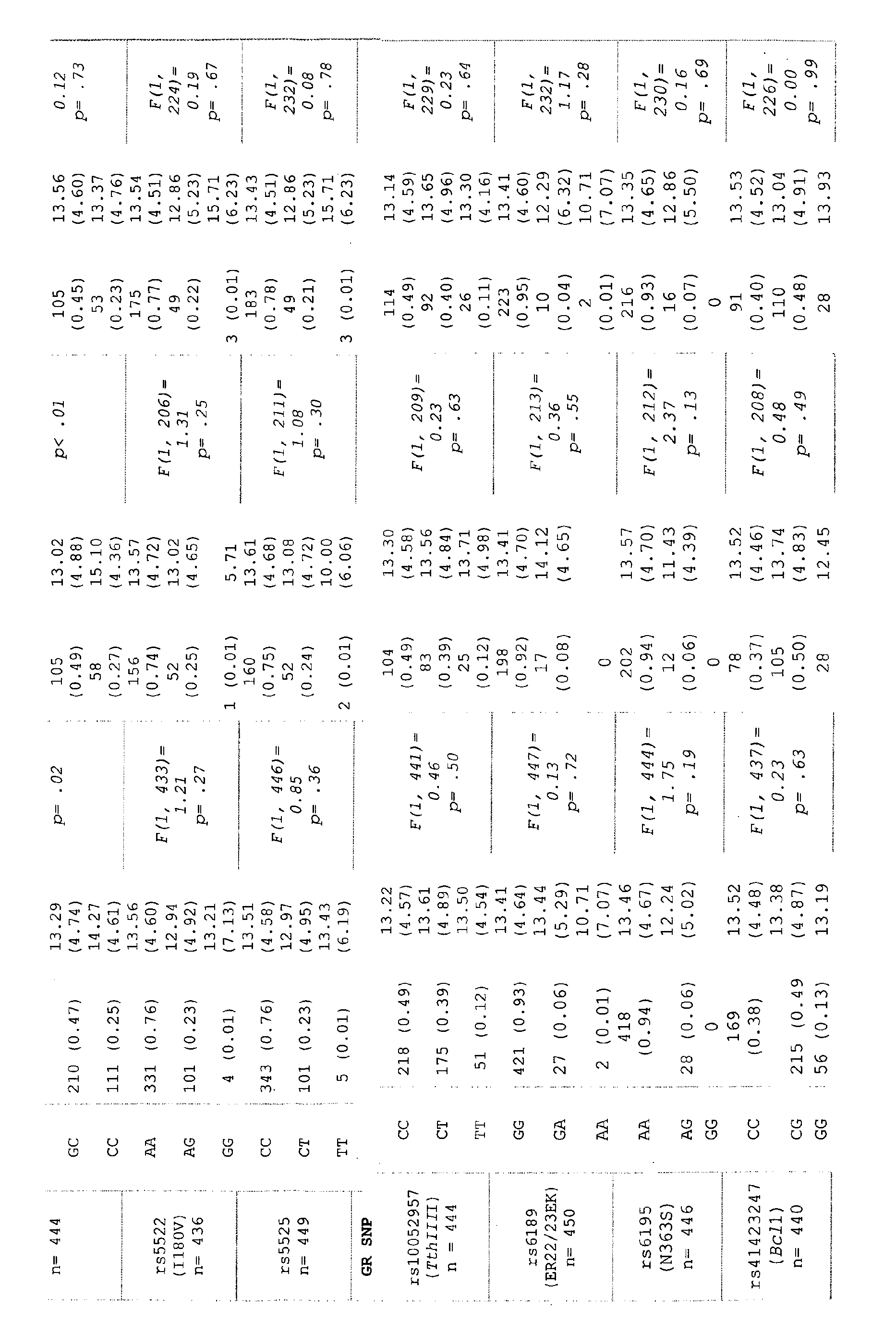

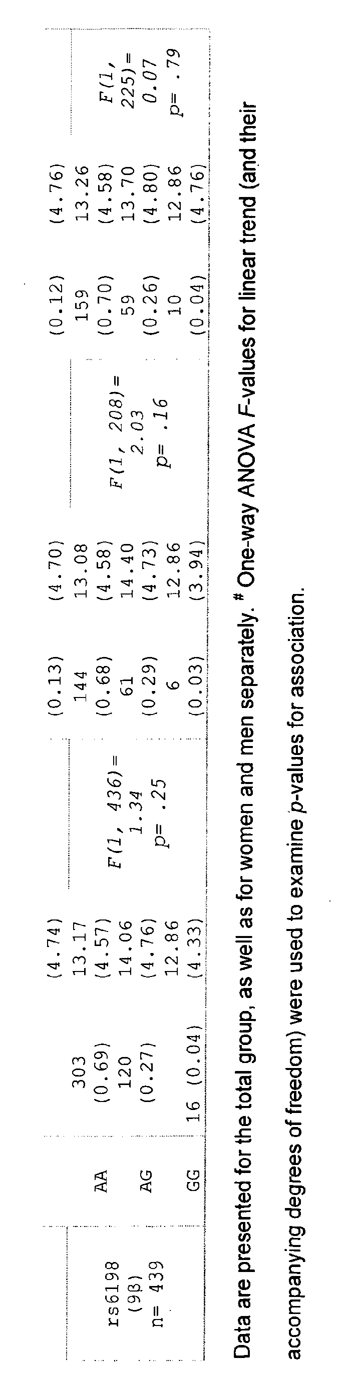

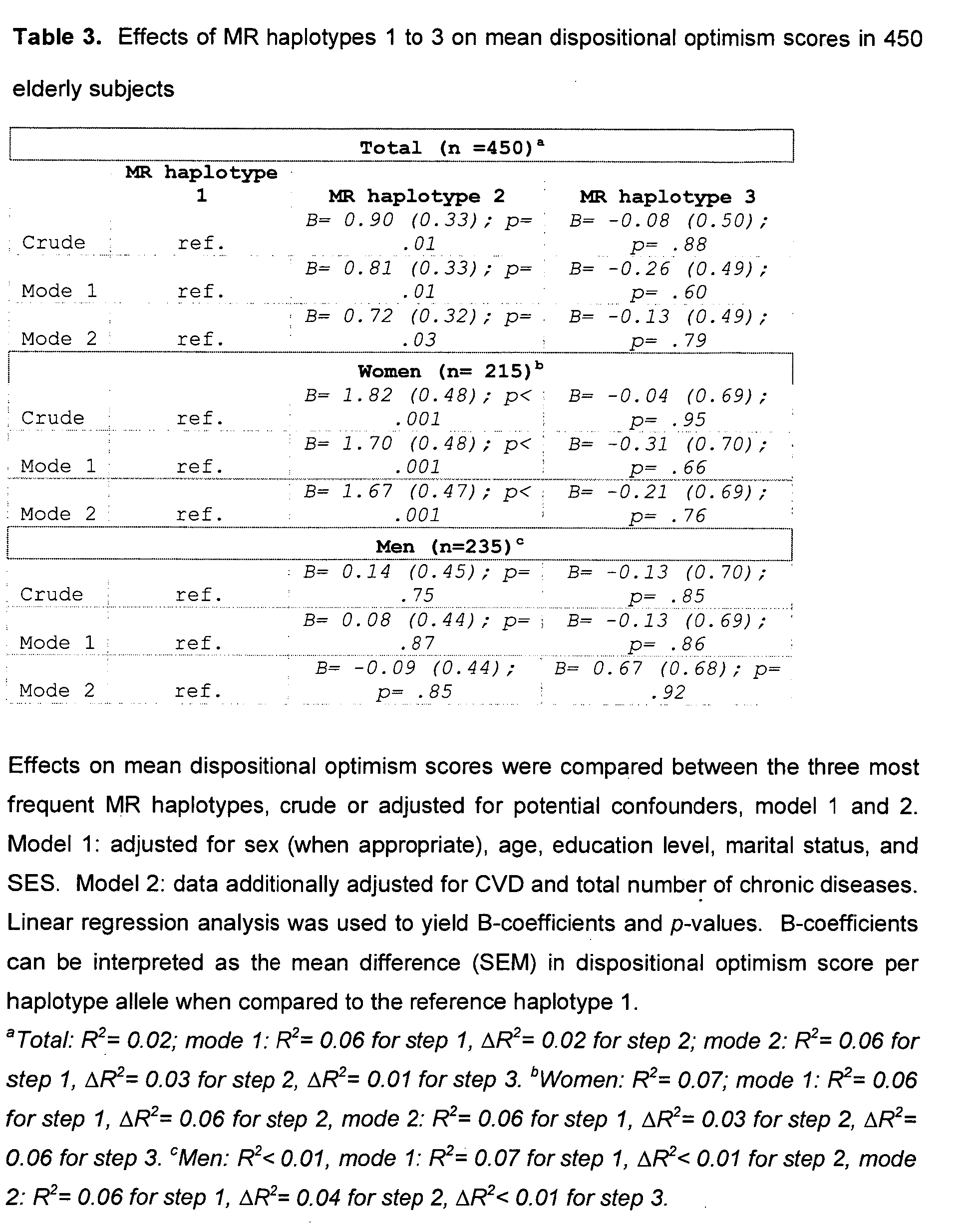

### Discussion

We found that the MR haplotype 2, which consists of the C-allele of the functional -2 G/C SNP and extends into the promoter region, was highly significant associated with higher levels of dispositional optimism in elderly women but not in men. This was independent of several potential confounders. Importantly, we were also able to show that haplotype 2 was associated with optimism in a dose dependent manner, with women having a 2/2 diplotype reporting even higher optimism scores than women with only one haplotype 2 allele. No effect was found for the GR haplotypes. This is the first report on a MR gene variant that is associated with a positive psychological trait in humans.

MR haplotype 2 contains the functional -2 G/C SNP. The C-allele of this SNP increases MR expression and MR-driven gene transcription *in vitro* [Nienke PNE09 in press] In addition, the -2 C-allele has been shown to associate with lower basal cortisol levels in elderly (Kuningas, de Rijk *et al* (2007)). Together with our results, this finding seems to fit with a study showing that higher levels of optimism associate with lower basal cortisol levels (Lai, Evans *et al* (2005)). It would be interesting to know whether the differences in optimism scores we found were also associated with variances In cortisol levels. Unfortunately, no cortisol data were assessed in the Arnhem elderly study. Furthermore. our results are also in line with a report showing that the MR 160 V-allele, or haplotype 3, associated with more depressive symptoms in a Dutch elderly cohort, the Leiden 85+ cohort (Kuningas, de Rijk *et al* (2007)). In our study this haplotype 3 (although carried by only 2 subjects) was associated with the lowest scores for optimism.

Only in women, MR haplotype 2 associated with higher levels of dispositional optimism. Sex differences have previously been reported for HPA responses to stress but also for personality traits (Kudielka and Kirschbaum (2005); Schmitt, Realo *et al* (2008)). A gene-by-sex interaction could contribute to this and indeed has been found for HPA axis functioning and personality (Lang, Hellweg *et al* (2008); Wust. Kumsta *et al* (2009)). Additionally, also in rodents sex-specific effects of genes are found, for example for the MR and its influence on behavioural stress response (Rozeboom, Akil *et al* (2007)). One of the possible explanations for this gene-by-sex interaction of the MR may be its interaction with sex steroids. Estrogens and progesterone modulate protein and mRNA expression of corticosteroid receptors (Castren, Patchev *et al* (1995); Turner (1997)). In addition, progesterone can also bind to the human MR (Quinkler, Meyer *et al* (2002)). However, all women were 65+ of age, which means they probably all have low levels of estradiol due to menopause. Still, when conducting certain cognitive tests, only in elderly women variability in endogenous estradiol levels has been reported to relate to differences in performance (Wolf and Kirschbaum (2002)).

No relation was found between the GR gene variants and optimism. Rodent studies have shown that both the MR and GR modulate anxiety- and depressive like behaviours, including learned helplessness (Urani, Chourbaji et al (2005); Rozeboom, Akil *et al* (2007)). Moreover, both the MR and GR are involved in behaviour and cognition-However, it seems that it is mainly the MR that is mediating choice of behavioural strategy, flexibility and reactivity (Oitzl and de Kloet (1992); Berger, Wolfer *et al* (2006); Brinks, van der Mark *et al* (2007)). When, for example after a training session rats are treated with a MR antagonist, they show an altered search-escape strategy in the Morris water maze. Blocking the GR had no such effect (Oitzl and de Kloet (1992)). To our knowledge there is only one study that reported an effect of MR blockage on cognitive flexibility in humans (Otte, Moritz *et al* (2007)). The importance of the GR for cognitive functioning during elevated levels of cortisol is generally accepted, but additional studies are warranted to elucidate the specific roles of the MR and GR in cognitive flexibility, coping behaviour and psychological traits.

To the best of our knowledge, this is the first study reporting on a gene variant that was associated with variability in the positive psychological trait dispositional optimism. Evidence is accumulating for optimism having influence on goal engagement and coping behaviour, Indirectly enhancing a multitude of health outcomes, (Scheier, Weintraub *et al* (1986); Plomin, Scheier *et al* (1992); Scheier and Carver (1992); Carver. Pozo *et al* (1993); Scheier, Carver *et al* (1994); Vickers and Vogeltanz (2000); Giltay, Geleijnse *et al* (2004); Giltay, Kamphuis *et al* (2006): Giltay, Zitman *et al* (2006); Nes and Segerstrom (2006); Geers, Wellman *et al* (2009)). Hopelessness, on the other hand, has been reported to increase risk for disease and mortality and is positively associated to stress-related disorders like depression (Everson, Goldberg *et al* (1996); Joiner, Steer *et al* (2001)). Optimists seem more resilient against everyday challenges. People with high levels of optimism are better in tolerating stressful conditions and choose a coping strategy that is appropriate for the situation. For example, in a study following women that were diagnosed with breast cancer, the more optimistic women were able to accept their situation and used positive reframing and also humour to deal with it, leading to less distress (Carver, Pozo *et al* (1993)). Optimists are cognitively more flexible, seek and perceive more social support, and more often turn to religion or exercise (Scheier and Carver (1992); Carver, Pozo *et al* (1993); Scheier, Carver *et al* (1994); Southwick, Vythilingam *et al* (2005)). Moreover, optimists cope better maybe in part because they perceive information from their environment differently. Optimists are able to ignore negative stimuli better when it is not relevant and have more attention to positive stimuli (Isaacowitz (2005)). The identification of genes and biological mechanisms underlying traits that confer resilience against stress could provide important information for pharmaco- and cognitive therapy in patients with anxiety- and depressive disorders. The mechanism by which glucocorticoids and the MR affect optimism remains undear. It has been postulated that people who are able to remain optimistic during challenging situations have a neurobiological system for reward and motivation that is hyperactive or resistant to change (Southwick, Vythilingam *et al* (2005)). Multiple studies have reported that glucocorticoids act on the brain reward system. An example is the effect of glucocorticoids on the motivation to take drugs, that is known to be mediated at least by the GR (Ambroggi, Turiault *et al* (2009)). Whether the MR is implicated in reward mechanisms needs further investigation.

We found an association between a MR gene variant and variability in optimism among elderly subjects. Multiple studies have reported on changes in emotional and cognitive functioning among older adults. Levels of optimism and positive effect but also cognitive functioning slowly decrease, while the prevalence of depressive symptoms and depressive disorder increases (de Beurs, Comijs *et al* (2005); Giltay, Zirman *et al* (2006); Kuningas, de Rijk *et al* (2007)). Malfunctioning of the HPA axis might be one of the underlying mechanisms. Expression of corticosteroid receptors in the brain changes during development, throughout adulthood and during aging (van Eekelen, Rots *et al* (1992); Schmidt, Enthoven *et al* (2003); Dalm, Enthoven *et al* (2005)). For example, expression of MR in the hippocampus is decreased in old rats. It is possible that MR gene variants play a modulating role, resulting in more or less decrease in MR expression, eventually leading to better or worse psychological functioning. As mentioned before, the -2 C-allele results in more expression of MR and a higher gene transactivation in vitro. Moreover, haplotype 2 also consists of two SNPs located in the promoter region for which the highest significant associations were found with optimism. It is very well possible that these SNPs have an additional and maybe even stronger effect on MR expression. Therefore, these promoter SNPs need to be tested for their effect on promoter activity.

To conclude, we found that the MR haplotype 2, including the functional -2 C-allele but not the 180 V-allele, was associated with higher levels of dispositional optimism in Dutch elderly females in a dose dependent manner. The results indicate that the MR modulates not only neuroendocrine and autonomic response to a stressor but can also affect a positive psychological trait, which may determine resilience against stress and depression.

### References

Ambroggi, F., M. Turiault, et al (2009) "Stress and addiction: glucocorticoid receptor in dopaminoceptive neurons facilitates cocaine seeking." Nat Neurosci 12(3): 247-9.
Barrett, J. C., B. Fry, et al (2005) "Haploview: analysis and visualization of LD and haplotype maps." Bioinformatics 21(2): 263-5.
Berger, S., D. P. Wolfer, et al (2006) "Loss of the limbic mineralocorticoid receptor impairs behavioral plasticity." Proc Natl Acad Sci USA 103(1) 195-200.
Brinks, V., M. H. van der Mark, et al (2007) "Differential MR/GR activation in mice results in emotional states beneficial or impairing for cognition." Neural Plast 2007: 90163.
Carver, C. S. and J- Connor-Smith (2009) "Personality and Coping." Annu Rev Psychol.
Carver, C. S., C. Pozo, et al (1993) "How coping mediates the effect of optimism on distress: a study of women with early stage breast cancer." J Pers Soc Psychol 65(2): 375-90.
Castren, M., V. K. Patchev, et al (1995) "Regulation of rat mineralocorticoid receptor expression in neurons by progesterone." Endocrinology 138(9): 3800-6.
Conrad, C. D., S. J. Lupien, et al (1997) "The effects of type 1 and type II corticosteroid receptor agonists on exploratory behavior and spatial memory in the Y-maze." Brain Res 759(1): 76-83.
Dalm, S., L Enthoven, et al (2005) "Age-related changes in hypothalamic-pituitary-adrenal axis activity of male C57BL/6J mice." Neuroendocrinology 81(6): 372-80.
de Beurs, E., H. Comijs, et al (2005) "Stability and change of emotional functioning in late life: modelling of vulnerability profiles." J Affect Disord 84(1) 53-62.
de Kloet, E. R., S. A. Van Acker, et al (2000) "Brain mineralocorticoid receptors and centrally regulated functions." Kidney Int 57(4): 1329-36.
De Kloet, E. R., E. Vreugdenhil, et al (1998) "Brain corticosteroid receptor balance in health and disease." Endocr Rev 99(3): 269-301.
Derijk, R. H., N. van Leeuwen, et al (2008) "Corticosteroid receptor-gene variants: modulators of the stress-response and implications for mental health." Eur J Pharmacol 585(2-3): 492-501.
DeRijk, R. H., S. Wust, et al (2006) "A common polymorphism in the mineralocorticoid receptor modulates stress responsiveness." J Clin Endocrinol Metab 91(12): 5083-9.
Everson, S. A., D. E. Goldberg, et al (1996) "Hopelessness and risk of mortality and incidence of myocardial infarction and cancer." Psychosom Med 58(2): 113-21.
Geers, A. L., J. A. Wellman, et al (2009) "Dispositional optimism and engagement: the moderating influence of goal prioritization." J Pers Soc Psychol 96(4): 913-32.
Giltay, E. J., J. M. Geleljnse, et al (2004) "Dispositional optimism and all-cause and cardiovascular mortality in a prospective cohort of elderly dutch men and women." Arch Gen Psychiatry 61(11): 1126-35*.*
Giltay, E. J., M. H. Kamphuis, et al (2006) "Dispositional optimism and the risk of cardiovascular death: the Zutphen Elderly Study." Arch intern Med 166(4): 431-6.
Giltay, E. J., F. G. Zifman, et a/ (2006) "Dispositional optimism and the risk of depressive symptoms during 15 years of follow-up: the Zutphen Elderly Study." J Affect Disord 91 (1): 45-52.
Isaacowitz, D. M. (2005) "The gaze of the optimist" Pers Soc Psychol Bull 31(3): 407-15.
Joiner, T. E, Jr., R. A. Steer, et al (2001) "Hopelessness depression as a distinct dimension of depressive symptoms among clinical and non-clinical samples." Behav Res Ther 39(5): 523-36.
Kudielka, B. M. and C. Kirschbaum (2005) "Sex differences in HPA axis responses to stress: a review." Biol Psychol 69(1): 113-32.
Kumsta, R., S. Entringer, et al (2007) "Sex specific associations between common glucocorticoid receptor gene variants and hypothalamus-pituitary-adrenal axis responses to psychosocial stress " Biol Psychiatry 62(8): 863-9.
Kuningas, M., R. H. de Rijk. et al (2007) "Mental performance in old age dependent on cortisol and genetic variance In the mineralocorticoid and glucocorticoid receptors." Neuropsychopharmacology 32(6): 1295-301.
Lai, J: C., P. D. Evans, et al (2005) optimism, positive affectivity, and salivary cortisol." Br J Health Psychol 10(Pt 4): 467-84.
Lang, U. E., R. Hellweg, et al (2008) "Gender-dependent association of a functional NGF polymorphism with anxiety-related personality traits." Pharmacopsychiatry 41(5): 196-9.
Lupien, S. J., C. W. Wilkinson, et al (2002) "The modulatory effects of corticosteroids on cognition: studies in young human populations." Psychoneuroendocrinology 27(3): 401-16.
Nes, L S. and S. C. Segerstrom (2006) "Dispositional optimism and coping: a meta-analytic review." Pers Soc Psychol Rev 10(3): 235-51.
Oitzl, M. S. and E. R. de Kloet (1992) "Selective corticosteroid antagonists modulate specific aspects of spatial orientation learning." Behav Neurosci 106(1): 62-71.
Otte, C., S. Moritz, et al (2007) "Blockade of the mineralocorticoid receptor in healthy men: effects on experimentally induced panic symptoms, stress hormones, and cognition." Neuropsychopharmacology 32(1) 232-8.
Plomin. R. M. F. Scheier, et al (1992) "Optimism, pessimism and mental health: a twin/adoption analysis" Person. individ. Diff 13(8): 921-930.
Quinkler, M., B. Meyer, et al (2002) "Agonistic and antagonistic properties of progesterone metabolites at the human mineralocorticoid receptor." Eur J Endocrinol 146(6): 789-99.
Rozeboom, A. M., H. Akil, et al (2007) "Mineralocorticoid receptor overexpression in forebrain decreases anxiety-like behavior and alters the stress response in mice." Proc Natl Acad Sci USA 104(11): 4688-93.
Scheier, M. F. and C. S. Carver (1985) "Optimism, coping, and health: assessment and implications of generalized outcome expectancies." Health Psychol 4(3): 219-47,
Scheier. M. F. and C. S. Carver (1992) "Effects of Optimism on Psychological and Physical Well-Being: Theoretical Overview and Empirical Update." Cognit Therapy Res 16(2): 201-228.
Scheier, M. F., C. S. Carver, et al (1994) "Distinguishing optimism from neuroticism (and trait anxiety, self-mastery, and self-esteem): a reevaluation of the Life Orientation Test" J Pers Soc Psychol 67(6): 1063-78.
Scheier, M. F., J. K Weintraub, et al (1986) Coping with stress: divergent strategies of optimists and pessimists." J Pers Soc Psycho/ 51(6): 257-64.
Schmidt, M. V., L. Enthoven, et al (2003) "The postnatal development of the hypothalamic-pituitary-adrenal axis in the mouse." Int J Dev Neurosci 21(3): 125-32.
Schmitt, D. P., A. Realo, et al (2008) "Why can't a man be more like a woman? Sex differences in Big Five personality traits across 55 cultures." J Pers Soc Psychol 94(1): 188-82,
Southwick, S. M., M. Vythilingam, et al (2005) "The psychobiology of depression and resilience to stress: implications for prevention and treatment." Annu Rev Clin Psychol 1: 255-91.
Steptoe, A., K. O'Donnell, et al (2008) "Neuroendocrine and inflammatory factors associated with positive affect in healthy men and women: the Whitehall II study." Am J Epidemiol 167(1): 98-102.
Tempelman, C. J. J. (1987). "Welbevinden bij ouderen: konstruktie van een meetinstrument [Well-being in the elderly: development of the Scale Subjective Well-being Older Persons] [dissertation]. Groningen, the Netherlands: University of Groningen; "
Turner, B. B. (1997) "Influence of gonadal steroids on brain corticosteroid receptors: a minireview." Neurochem Res 22(11): 1375-85.
Urani, A., S. Chourbaji, et al (2005) "Mutant mouse models of depression: candidate genes and current mouse lines." Neurosci Biobehav Rev 29(4-5): 805-28.
van den Hombergh, C. E., E. G. Schouten, et al (1995) "Physical activities of noninstitutionalized Dutch elderly and characteristics of inactive elderly." Med Sci Sports Exerc 27(3) 334-9.
van Eekelen, J. A., N. Y. Rots, et al (1992) "The effect of aging on stress responsiveness and central corticosteroid receptors in the brown Norway rat." Neurobiol Aging 13(1) 159-70.
Vickers, K. S. and N. D. Vogeltanz (2000) "Dispositional optimism as a predictor of depressive symptoms over time" Person. lndivid. Diff. 28: 259-272.
Wolf, O. T. and C. Kirschbaum (2002) "Endogenous estradiol and testosterone levels are associated with cognitive performance in older women and men." Horm Behav 41(3) 259-66.
Wust, S., R. Kumsta, et al (2009) "Sex-specific association between the 5-HTT gene-linked polymorphic region and basal cortisol secretion." Psychoneuroendocrinology*.*

### EXAMPLE 2: Human mineralocorticoid receptor gone variants modulate cognitive vulnerability for depression

The mineralocorticoid receptor (MR) plays a central role in the regulation of hypothalamic-pituitary-adrenal (HPA) axis, activity. Animal studies indicate that the MR mediates effects of cortisol on emotions and coping behaviour. We hypothesise that human MR-gene variants influence cognition and emotions. We have identified a MR haplotype (frequency 36) to relate to higher dispositional optimisim in elderly women, not in men (see Example 1).

In the present study 154 students (46 M/108 F; 23.9±5 yrs) completed a questionnaire that measures cognitive vulnerability to depression and that includes subscales for hopelessness, rumination and aggression (LEIDS-R- see Appendix; Van der Dose, Behav Res & Therap 40:105-120, 2002); Leiden Index of Depression Sensitivity-Revised). Neuroticism was also measured (NEO-PI) as well as symptoms of depression (HADS-D). MR SNPs and haplotypes were assessed, resulting in three haplotypes with frequencies of .50; .35; .13.

Significant associations were found only in females between the haplotype with a frequency .35 and lower scores for hopelessness, aggression, risk aversion, neuroticism (*p*< .05), and in particular for rumination (p= .001), persisting after adjustment for age and emotional abuse during childhood. Excluding currently depressed participants (n= 14) strengthened the results. Moreover, this haplotype significantly associated with less symptoms of depression (*p*< .05). The results fit with our study showing an association between this haplotype and higher dispositional optimism (Example 1), also only in women. Together the data indicate that MR-gene variants modulate cognitive vulnerability for depression.

In the present study 154 Dutch students (46 M/108 F; 23.9±5 yrs) completed a questionnaire that was prepared to assess cognitive vulnerability to depression, the LEIDS-R (Leiden Index of Depression Sensitivity-Revised). This questionnaire measures cognitive reactivity to sad mood (Van der Does, A.J.W., 2002). The subjects have to read the instructions and Indicate to what extend they agree with in total 34 statements. The scale includes 6 subscales, namely hopelessness/suicidality, acceptance/coping, aggression, contro/perfectionism, risk aversion, and rumination. In addition, neuroticism was measured (NED-PI) as well as symptoms of depression and anxiety (HADES-D and HADS-A). All students were genotyped for the MR SNPs -2G/C (rs2070951) and l18UV (rs5522) and haplotypes were reconstructed.

Figures 6-11 show the results for the different scales (untransformed data) for the total group and for the females and males separately, excluding cases with current depression (n= 14). P-values represent Analysis Of Variance (ANOVA) results, without correction for confounding effects of sex (when appropriate), age and emotional abuse. In addition, dummy variables were created for the haplotypes 1-3, followed by linear regression analysis to test association between the haplotypes, while correcting for the covariates. For this analysis, data for the subscales Acceptance/Coping, Aggression, Perfectionism/Control and Hopelessness were transformed (Square Root) to approach a normal distribution.

### APPENDIX

### LEIDS-R Questionnaire

### Instructions

Below are a number of statements that may apply to you to a lesser or greater extent.

Almost every statement concerns your thoughts about a certain matter *at times when you feel down or when you are in a low mood.* This does not mean a seriously depressed mood or true depression. Your task is to indicate the extent to which the statements apply to you when you feel somewhat sad.

*Try* to *imagine the following situation when filling out this questionnaire.*

It is certainly not a good day, but you don't feel truly down or depressed. Perhaps your mood is an early sign of something worse to come, but things might also improve in the next day or two.

On a scale ranging from 0 to to (0 = not at all sad; 10 = extremely sad; 6 and above = a truly depressed mood), you would choose a 3 or 4 to describe your mood.

The scale looks like this:

| | | | | | | | | | | |
|---|---|---|---|---|---|---|---|---|---|---|
| 0 | 1 | 2 | 3 | ④ | 5 | 6 | 7 | 8 | 9 | 10 |
| **not at all sad** | | | | **somewhat sad** | | | **depressed** | | | **very depressed** |

Please try to imagine yourself in the above situation, for instance by thinking back to the last time you felt somewhat sad (score 3 or 4).

{Now take soma time to imagine such a situation.}

*To what extent* are *you able* to *imagine such* a *situation?* ° swell
° somewhat
° not at all

Now proceed to the next question (even If you find it difficult to Imagine yourself in such a situation).

| | | **This applies to me .........: (please circle)** | | | | |
|---|---|---|---|---|---|---|
| | | ***not at all*** | ***a bit*** | ***mode-rately*** | ***strongly*** | ***very strongly*** |
| 1. | I can only think positive when I am in a good mood. | 0 | 1 | 2 | 3 | 4 |
| 2. | When in a low mood, I take fewer risks. | 0 | 1 | 2 | 3 | 4 |
| 3. | When I feel sad, I spend more time thinking about what my moods reveal about me as a person. | 0 | 1 | 2 | 3 | 4 |
| 4. | When in a sad mood, I am more creative than usual. | 0 | 1 | 2 | 3 | 4 |
| 5. | When I feel down, more often feel hopeless about everything. | 0 | 1 | 2 | 3 | 4 |
| 6. | When I feel down, I am more busy trying to keep Images and thoughts at bay. | 0 | 1 | 2 | 3 | 4 |
| 7. | In a sad mood, I do more things that I will later regret | 0 | 1 | 2 | 3 | 4 |
| 8. | When feel sad, I go out and do more pleasurable activities. | 0 | 1 | 2 | 3 | 4 |
| 9. | When I feel sad, I feel as if I care less if I lived or died. | 0 | 1 | 2 | 3 | 4 |
| 10. | When I feel sad, am more helpful. | 0 | 1 | 2 | 3 | 4 |
| 11. | When I feel sad, I am less inclined to express disagreement with someone else. | 0 | 1 | 2 | 3 | 4 |
| 12. | When I feel somewhat depressed, I think I can permit myself fewer mistakes. | 0 | 1 | 2 | 3 | 4 |
| 13. | When I feel dawn, I more often feel overwhelmed by things. | 0 | 1 | 2 | 3 | 4 |
| 14. | When in a low mood. I am more inclined to avoid difficulties or conflicts. | 0 | 1 | 2 | 3 | 4 |
| 15. | When I feel down, I have a better intuitive feeling for what people really mean. | 0 | 1 | 2 | 3 | 4 |
| 16. | When In a sad mood, I become more bothered by perfectionism. | 0 | 1 | 2 | 3 | 4 |
| 17. | When 1 feel sad, I more often think that I can make no one happy. | 0 | 1 | 2 | 3 | 4 |
| 18. | When I feel bad, I feel more like breaking things. | 0 | 1 | 2 | 3 | 4 |
| 19. | I work harder when I feel down. | 0 | 1 | 2 | 3 | 4 |
| 20. | When I feel sad, I feel less able to cope with everyday tasks and interests. | 0 | 1 | 2 | 3 | 4 |
| | | ***not at all*** | ***a bit*** | ***mode-rately*** | ***strongly*** | ***very strongly*** |

| | | ***not at all*** | ***a bit*** | ***mode-rately*** | ***strongly*** | ***very strongly*** |
|---|---|---|---|---|---|---|
| 21. | in a sad mood, I am bothered more by aggressive thoughts. | 0 | 1 | 2 | 3 | 4 |
| 22. | When I feel down, I more easily become cynical (blunt) or sarcastic. | 0 | 1 | 2 | 3 | 4 |
| 23. | When I feel down, I feel more like escaping everything. | 0 | 1 | 2 | 3 | 4 |
| 24. | When in a sad mood, I feel more like myself. | 0 | 1 | 2 | 3 | 4 |
| 25. | When I feel down, more often neglect things, | 0 | 1 | 2 | 3 | 4 |
| 26. | When I feel sad, I do more risky things. | 0 | 1 | 2 | 3 | 4 |
| 27. | When I am sad, I have more problems concentrating. | 0 | 1 | 2 | 3 | 4 |
| 28. | When in a low mood, I am nicer than usual. | 0 | 1 | 2 | 3 | 4 |
| 29. | When I feel down, I lose my temper more easily. | 0 | 1 | 2 | 3 | 4 |
| 30. | When I feel sad, I feel more that people would be better off If were dead. | 0 | 1 | 2 | 3 | 4 |
| 31. | When I feel down, am more inclined to want to keep everything under control. | 0 | 1 | 2 | 3 | 4 |
| 32. | When I feel sad, I spend more time thinking about the possible causes of my moods. | 0 | 1 | 2 | 3 | 4 |
| 33. | When In a sad mood, I more often think about how my life could have been different | 0 | 1 | 2 | 3 | 4 |
| 34. | When I feel sad, more thoughts of dying or harming myself go through my mind. | 0 | 1 | 2 | 3 | 4 |
| | | ***not at all*** | ***a bit*** | ***mode-rately*** | ***strongly*** | ***very strongly*** |

### Statistical analysis of LEIDS-R questionnaire results

COMPUTE HOP = MEAN.4(leids5,leids9,leids17,leids30,leids34) * 5.

COMPUTE ACC = MEAN.4(leids4,leids1 0,leids15,leids24,leids28)* 5.

COMPUTE AGG = MEAN.5(leids7,leids18,leids21,leids22,leids26,leids29) * 6.

COMPUTE CON = MEAN.5(leids3,leids8,leids12,leids16,leids19,leids31) * 6 .

COMPUTE RAV = MEAN.5(leids1,leids2,leids6,leids11,leids14,leids23)* 6.

COMPUTE RUM = MEAN.5(leids13,leids20,leids25,leids27,leids32,leids33)* 6.

EXECUTE.

COMPUTE LEIDSR = HOP + ACC + AGG + CON + RAV + RUM.

EXECUTE.

In this syntax, subscales are computed with the MEAN function and multiplied by the number of items.

Of course, this is the same as summing the items - if there are no missing values.

This syntax allows one missing item per subscale (the missing value is replaced with the average item score for that particular subscale),

Labels:
HOP = hopelessness/suicidality
ACC = acceptance/coping
AGG = aggression
CON = control/perfectionism
HAV = risk aversion
RUM = rumination

### EMBODIMENTS OF INVENTION

1. A method of assessing the susceptibility of a subject to an anxiety disorder or depression, the method comprising genotyping any one or more single nucleotide polymorphisms (SNPs) selected from the group consisting of rs3216799, rs6814934, rs7658048, rs2070950 and rs2070951, and/or one or more polymorphic sites which are in linkage disequilibrium with any one or more SNPs selected from the group consisting of rs3216799, rs6814934, rs7658048, rs2070950 and rs2070951 wherein reduced susceptibility is indicated when the allele of the one or more SNPs is respectively one or more of '+CT', 'C', 'T', `C' and 'C', and/or when the allele of the one or more polymorphic sites is one that is in linkage disequilibrium with the respective one or more '+CT, 'C', 'T', 'C' and 'C' alleles of the one or more SNPs.
2. A method according to Embodiment 1, wherein genotyping any one or more SNPs selected from the group consisting of rs3216799, rs6814934, rs7658048, rs2070950 and rs2070951, and/or one or more polymorphic sites which are in linkage disequilibrium with any one or more SNPs selected from the group consisting of rs3216799, rs6814934, rs7658048, rs2070950 and rs2070951, comprises contacting a sample of nucleic acid from the subject with one or more nucleic acid molecules that hybridise selectively to a genomic region encompassing any one or more SNPs selected from the group consisting of rs3216799, rs6814934, rs7656048, rs2070950 and rs2070951, and/or a genomic region encompassing one or more polymorphic sites which are in linkage disequilibrium with any one or more SNPs selected from the group consisting of rs3216799, rs6814934, rs7658048, rs2070950 and rs2070951.
3. A method according to Embodiment 1 or 2, wherein the subject is a female human.
4. A method according to any of Embodiments 1-3, wherein the one or more polymorphic sites which are in linkage disequilibrium with any one or more SNPs selected from the group consisting of rs3216799, rs6814934, rs7656048, rs2070950 and rs2070951, are within the mineralocorticoid receptor (MR) gene.
5. A method according to any of Embodiments 1-4, wherein the one or more polymorphic sites which are in linkage disequilibrium with any one or more SNPs selected from the group consisting of rs3216799, rs6814934, rs7658048, rs2070950 and rs2070951, are SNPs selected from the group consisting of rs5522, rs5525 and rs7671250, wherein reduced susceptibility is indicated when the allele of one or more of rs5522, rs5525 and rs7671250 is respectively 'A', 'C' and 'T'.
6. A method according to any of Embodiments 1-5, wherein the one or more polymorphic sites are SNPs selected from the group consisting of rs7671250, rs5522, rs5525, rs4835519, rs2172002, rs11929719, rs11099695, rs11730626, rs2070949, rs2248038, rs9992256, rs5520 and SNP x at position 149585620 In the MR gene as numbered in Figure 4.
7. A method according to any of Embodiments 1-6, wherein a further genetic locus associated with an anxiety disorder or depression is analysed in the subject
8. A method according to Embodiment 7, wherein the further genetic locus is any one or more of the glucocorticoid receptor (GR) gene, a heat shock protein gene, the P-glycoprotein gene and the corticotropin releasing hormone receptor-1 (CRHR-1) gene.
9. A method according to any of Embodiments 1-8, wherein one or more of the age, sex, body mass index (BMI), smoking status, childhood trauma status, or stress status of the subject is considered.
10. A method according to Embodiment 2, wherein the sample of nucleic acid from the subject is subjected to a nucleic acid amplification before contacting with one or more nucleic add molecules that hybridise selectively to the any one or more SNPs selected from the group consisting of rs3216799, rs6814934, rs7658048, rs2070950 and rs2070951, and/or to one or more polymorphic sites which are in linkage disequilibrium with any one or more SNPs selected from the group consisting of rs3216799, rs6814934, rs7658048, rs2070950 and rs2070951.
11. Use of one or more nucleic acid molecules that hybridise selectively to a genomic region encompassing any one or more SNPs selected from the group consisting of rs3216799, rs6814934, rs7658048, rs2070950 and rs2070951, and/or to a genomic region encompassing one or more polymorphic sites which are in linkage disequilibrium with any one or more SNPs selected from the group consisting of rs3216799, rs6814934, rs7658048, rs2070950 and rs2070951 for assessing the susceptibility of a subject to an anxiety disorder or depression, wherein reduced susceptibility is indicated when the allele of the one or more SNPs is respectively one or more of '+CT', 'C', 'T', 'C' and 'C', and/or when the allele of the one or more polymorphic sites is one that is in linkage disequilibrium with the respective one or more '+CT', 'C', 'T', 'C' and 'C' alleles of the one or more SNPs
12. One or more nucleic acid molecules that hybridise selectively to a genomic region encompassing any one or more SNPs selected from the group consisting of rs3216799, rs6814934, rs7658048, rs2070950 and rs2070951, and/or to a genomic region encompassing one or more polymorphic sites which are in linkage disequilibrium with any one or more SNPs selected from the group consisting of rs3216799, rs6814934, rs7658048, rs2070950 and rs2070951 for use in assessing the susceptibility of a subject to an anxiety disorder or depression, wherein reduced susceptibility is indicated when the allele of the one or more SNPs is respectively one or more of '+CT', 'C', 'T', 'C' and 'C', and/or when the allele of the one or more polymorphic sites is one that is in linkage disequilibrium with the respective one or more '+CT', 'C', 'T', 'C' and 'C' alleles of the one or more SNPs.
13. Use of one or more nucleic acid molecules that hybridise selectively to a genomic region encompassing any one or more SNPs selected from the group consisting of rs3216799, rs6814934, rs7658048, rs2070950 and rs2070951, and/or to a genomic region encompassing one or more polymorphic sites which are in linkage disequilibrium with any one or more SNPs selected from the group consisting of rs3216799, rs6814934, rs7658048, rs2070950 and rs2070951 in the manufacture of a reagent for assessing the susceptibility of a subject to an anxiety disorder or depression, wherein reduced susceptibility is indicated when the allele of the one or more SNPs is respectively one or more of '+CT, 'C', 'T', 'C' and `C', and/or when the allele of the one or more polymorphic sites is one that is in linkage disequilibrium with the respective one or more '+CT', 'C', `T', 'C' and 'C' alleles of the one or more SNPs.
14. A kit of parts for use in assessing the susceptibility of a subject to an anxiety disorder or depression, the kit comprising one or more nucleic acid molecules that hybridise selectively to a genomic region encompassing any two or more SNPs selected from the group consisting of rs3216799, rs6814934, rs7668048, rs2070950 and rs2070951, and/or that hybridise selectively to a genomic region encompassing two or more polymorphic sites in linkage disequilibrium with any one or more SNPs selected from the group consisting of rs3216799, rs6814934, rs7658048, rs2070950 and rs2070951.
15. A kit of parts for use in assessing the susceptibility of a subject to an anxiety disorder or depression, the kit comprising one or more nucleic acid molecules that hybridise selectively to a genomic region encompassing any one or more SNPs selected from the group consisting of rs3216799, rs6814934, rs7658048. rs2070950 and rs2070951 and that hybridise selectively to a genomic region encompassing one or more polymorphic sites in linkage disequilibrium with any one or more SNPs selected from the group consisting of rs3216799, rs6814934, rs7658048, rs2070950 and rs2070951.
16. A solid substrate for use in assessing the susceptibility of a subject to an anxiety disorder or depression, the solid substrate having attached thereto one or more nucleic acid molecules that hybridise selectively to a genomic region encompassing any two or more SNPs selected from the group consisting of rs3216799, rs6814934, rs7658048, rs2070950 and rs2070951, and/or that hybridise selectively to a genomic region encompassing two or more polymorphic sites in linkage disequilibrium with any one or more SNPs selected from the group consisting of rs3216799, rs6814934, rs7658048, rs2070950 and rs2070951.
17. A solid substrate for use in assessing the susceptibility of a subject to an anxiety disorder or depression, the solid substrate having attached thereto one or more nucleic acid molecules that hybridise selectively to a genomic region encompassing any one or more SNPs selected from the group consisting of rs3216799, rs6814934, rs7658048, rs2070950 and rs2070951, and that hybridise selectively to a genomic region encompassing one or more polymorphic sites in linkage disequilibrium with any one or more SNPs selected from the group consisting of rs3216799, rs6814934, rs7658048, rs2070950 and rs2070951.
18. A kit of parts according to Embodiment 14 or 15, or solid substrate according to Embodiment 16 or 17, wherein the polymorphic sites in linkage disequilibrium with any one or more SNPs selected from the group consisting of rs3216799, rs6814934, rs7658048, rs2070950 and rs2070951, are SNPs selected from the group consisting of rs5522, rs5525 and rs7671250.
19. A kit of parts according to Embodiment 14 or 15, or solid substrate according to Embodiment 16 or 17, wherein the polymorphic sites in linkage disequilibrium with any one or more SNPs selected from the group consisting of rs3216799, rs6814934, rs7658048, rs2070950 and rs2070951, are SNPs selected from the group consisting of rs7671250, rs5522, rs5525, rs4835519, rs2172002, rs11929719, rs11099695, rs17730626, rs2070949, rs2248038, rs9992256, rs6520 and SNP x at position 149585620 in the MR gene as numbered in Figure 4.
20. A kit of parts according to any of Embodiments 14, 15, 18 and 19, or a solid substrate according to any of Embodiments 16-19, further comprising a nucleic acid molecule that hybridises selectively to a further genetic locus associated with an anxiety disorder or depression.
21. A kit of parts or solid substrate according to Embodiment 20, wherein the further genetic locus is any one or more of the glucocorticoid receptor (GR) gene, a heat shock protein gene, the P-glycoprotein gene and the corticotropin releasing hormone receptor-1 (CRHR-1) gene.
22. A method of recording data on the susceptibility of a subject to an anxiety disorder or depression, the method comprising carrying out the method of any of Embodiments 1-10 and recording the results on a data carrier.
23. A method of preparing a data carrier containing data on the susceptibility of a subject to an anxiety disorder or depression, the method comprising carrying out the method of Embodiment 22.
24. A method according to Embodiment 22 or 23 wherein the data is recorded in electronic form.
25. A method of combating an anxiety disorder or depression in a subject, the method comprising assessing the susceptibility of a subject to an anxiety disorder or depression according to any of Embodiments 1-10 and depending upon the outcome of the assessment treating the subject.
26. A method according to Embodiment 25, wherein treating the subject comprises administering any one or more of an anti-depressant, an anti-convulsant, a beta-blocker, cortisol, a cortisol agonist, a cortisol antagonist, or an agent that modulates MR-expression to the subject
27. A compound for use in combating an anxiety disorder or depression In a subject who has been assessed as having, or having an increased likelihood of developing, an anxiety disorder or depression according to any of Embodiments 1-10, the compound being selected from an anti-depressant, an anti-convulsant, a beta-blocker, cortisol, a cortisol agonist, a cortisol antagonist, or an agent that modulates MR-expression.
28. Use of a compound in the manufacture of a medicament for combating an anxiety disorder or depression in a subject who has been assessed as having, or having an increased likelihood of developing, an anxiety disorder or depression according to any of Embodiments 1-10, the compound being selected from an anti-depressant, an anti-convulsant, a beta-blocker, cortisol, a cortisol agonist, a cortisol antagonist, or an agent that modulates MR-expression.
29. A method according to any of Embodiments 1-10 and 22-26, a use according to any of Embodiments 11, 13 and 28, a nucleic acid according to Embodiment 12, a kit of parts according to any of Embodiments 14, 15 and 18-21, a solid substrate according to any of Embodiments 16-21, and a compound according to Embodiment 27, wherein the anxiety disorder is any of substance-induced anxiety disorder, generalised anxiety, panic disorder, acute stress disorder, post traumatic stress disorder, adjustment disorder with anxious features, social phobia, obsessive-oompulsive disorder or specific phobias.
30. Any novel method of assessing susceptibility to an anxiety disorder or depression in a subject as herein disclosed.
31. Any novel kit of parts as herein disclosed.

## Claims

1. A method of assessing the susceptibility of a subject to an anxiety disorder or depression, the method comprising determining whether the subject has a haplotype comprising rs3216799, rs6814934, rs7658048, rs2070950 and rs2070951 with respective alleles '+CT', 'C', 'T', 'C' and 'C'.

2. A method according to Claim 1, wherein determining whether the subject has a haplotype comprising rs3216799, rs6814934, rs7658048, rs2070950 and rs2070951 with respective alleles '+CT', 'C', 'T', 'C` and 'C', comprises genotyping any one or more single nucleotide polymorphisms (SNPs) selected from the group consisting of rs3216799, rs6814934, rs7658048, rs2070950 and rs2070951, and/or one or more polymorphic sites which are in linkage disequilibrium with any one or more SNPs selected from the group consisting of rs3216799, rs6814934, rs7658048, rs2070950 and rs2070951, wherein reduced susceptibility is indicated when the allele of the one or more SNPs is respectively one or more of '+CT', 'C', 'T', 'C' and 'C', and/or when the allele of the one or more polymorphic sites is one that is in linkage disequilibrium with the respective one or more '+CT', 'C', T, 'C' and 'C' alleles of the one or more SNPs.

3. A method according to Claim 1 or 2, wherein the subject is a female human.

4. A method according to any of Claims 1-3, wherein the one or more polymorphic sites which are In linkage disequilibrium with any one or more SNPs selected from the group consisting of rs3216799, rs6814934, rs7658048, rs2070950 and rs2070951, are within the mineralocorticoid receptor (MR) gene.

5. A method according to any of Claims 1-4, wherein the one or more polymorphic sites which are in linkage disequilibrium with any one or more SNPs selected from the group consisting of rs3216789, rs6814934, rs7658048, rs2070950 and rs2070951, are SNPs selected from the group consisting of rs5522, rs5525 and rs7671250, wherein reduced susceptibility is indicated when the allele of one or more of rs5522, rs5525 and rs7671250 is respectively 'A', 'C' and 'T'.

6. A method according to any of Claims 1-5, wherein the one or more polymorphic sites are SNPs selected from the group consisting of rs7671250, rs5522, rs5525, rs4835519, rs2172002, rs11929719, rs11099695, rs11730626, rs2070949, rs2248038, rs9992256, rs5520, and SNP x at position 149585620 in the MR gene as numbered in Figure 4.

7. Use of one or more nucleic acid molecules that hybridise selectively to a genomic region encompassing any one or more SNPs selected from the group consisting of rs3216799, rs6814934, rs7658048, rs2070950 and rs2070951, and/or to a genomic region encompassing one or more polymorphic sites which are in linkage disequilibrium with any one or more SNPs selected from the group consisting of rs3216799, rs6814934, rs7658048, rs2070950 and rs2070951 for assessing the susceptibility of a subject to an anxiety disorder or depression, wherein reduced susceptibility is indicated when the allele of the one or more SNPs is respectively one or more of '+CT', 'C', 'T', 'C' and 'C', and/or when the allele of the one or more polymorphic sites is one that is in linkage disequilibrium with the respective one or more '+CT', 'C', 'T', 'C' and 'C' alleles of the one or more SNPs

8. One or more nucleic add molecules that hybridise selectively to a genomic region encompassing any one or more SNPs selected from the group consisting of rs3216799, rs6814934, rs7658048, rs2070950 and rs2070951, and/or to a genomic region encompassing one or more polymorphic sites which are in linkage disequilibrium with any one or more SNPs selected from the group consisting of rs3216799, rs6814934, rs7658048, rs2070950 and rs2070951 for use in assessing the susceptibility of a subject to en anxiety disorder or depression, wherein reduced susceptibility is indicated when the allele of the one or more SNPs Is respectively one or more of '+CT', 'C', 'T', 'C' and 'C', and/or when the allele of the one or more polymorphic sites is one that is in linkage disequilibrium with the respective one or more '+CT,' 'C', 'T', 'C' and 'C' alleles of the one or more SNPs.

9. A kit of parts or solid substrate for use in assessing the susceptibility of a subject to an anxiety disorder or depression, the kit comprising or the solid substrate having attached thereto one or more nucleic acid molecules that hybridise selectively to a genomic region encompassing any two or more SNPs selected from the group consisting of rs3216799, rs6814934, rs7658048, rs2070950 and rs2070951, and/or that hybridise selectively to a genomic region encompassing two or more polymorphic sites in linkage disequilibrium with any one or more SNPs selected from the group consisting of rs3216799, rs6814934, rs7658048, rs2070950 and rs2070951.

10. A kit of parts or solid substrate for use in assessing the susceptibility of a subject to an anxiety disorder or depression, the kit comprising or the solid substrate having attached thereto one or more nucleic acid molecules that hybridise selectively to a genomic region encompassing any one or more SNPs selected from the group consisting of rs3216799, rs6894934, rs7658048, rs2070950 and rs2070951, and that hybridise selectively to a genomic region encompassing one or more polymorphic sites in linkage disequilibrium with any one or more SNPs selected from the group consisting of rs3210799, rs6814934, rs7658048, rs2070950 and rs2070951.

11. A kit of parts or solid substrate according to Claim 9 or 10, wherein the polymorphic sites in linkage disequilibrium with any one or more SNPs selected from the group consisting of rs3216799, rs6814934, rs7658048, rs2070950 and rs2070951, are SNPs selected from the group consisting of rs5522, rs5525 and rs7671250, or are SNPs selected from the group consisting of rs7671250, rs5522, rs5525, rs4835519, rs2172002, rs11929719, rs11099695, rs11730626, rs2070949. rs2248038, rs9992256, rs5520 and SNP x at position 149585620 in the MR gene as numbered in Figure 4.

12. A method of combating an anxiety disorder or depression in a subject, the method comprising assessing the susceptibility of a subject to an anxiety disorder or depression according to any of Claims 1-6 and depending upon the outcome of the assessment treating the subject.

13. A method according to Claim 12, wherein treating the subject comprises administering any one or more of an anti-depressant, an anti-convulsant, a beta-blocker, cortisol, a cortisol agonist, a cortisol antagonist, or an agent that modulates MR-expression to the subject

14. A method according to any of Claims 1-6, 12 and 13, a use according to Claim 7, a nucleic acid according to Claim 8, a kit of parts according to any of Claims 9-11, and a solid substrate according to any of Claims 9-11, wherein the anxiety disorder is any of substance-induced anxiety disorder, generalised anxiety, panic disorder, acute stress disorder, post-traumatic stress disorder, adjustment disorder with anxious features, social phobia, obsessive-compulsive disorder or specific phobias.

15. An isolated polynucleotide comprising an MR gene sequence having a polymorphic site at position 149585620 as numbered in Figure 4.
